# EUROPÄISCHE PATENTANMELDUNG

(11) **EP 4 082 665 A1**
(43) Veröffentlichungstag der Anmeldung: **02.11.2022**
(21) Anmeldenummer: 21171551.1
(22) Anmeldetag: 30.04.2021
(51) Int. Cl.: B01L 3/02, G01N 35/10, G01N 35/00

(54) **SYSTEM MIT HANDGEHALTENER PIPETTIERVORRICHTUNG**

(71) Anmelder: Eppendorf SE, 22339 Hamburg (DE)
(72) Erfinder: RÜHMANN, Lisa Marie, 21256 Handeloh (DE); FÖRTHMANN, Benjamin, 22399 Hamburg (DE)
(74) Vertreter: Kirchner, Christian

(57) **Zusammenfassung**

Die Erfindung bezieht sich auf ein System zur Protokollierung einer Abfolge von Pipettiervorgängen, die Bestandteile einer Pipettiermethode sind, die in einem Laborexperiment zum Einsatz kommt, wobei das System eine Unterbrechung einer Methodenaufzeichnung und die Möglichkeit zu dessen Fortsetzung implementiert. Die Erfindung betriff auch ein Verfahren zur Verwendung des Systems.

## Beschreibung

Die Erfindung bezieht sich auf ein System mit mindestens einer handgehaltenen Pipettiervorrichtung, und ein Verfahren zu dessen Verwendung.

Solche handgehaltenen Pipettiervorrichtungen werden üblicherweise in medizinischen, biologischen, biochemischen, chemischen und anderen Laboratorien verwendet. Sie dienen im Labor zum Transport und Übertragen von fluiden Proben mit kleinen Volumina, insbesondere zur präzisen Dosierung der Proben. Zwei bekannten Klassen solcher handgehaltenen Pipettiervorrichtungen unterscheiden sich durch das jeweilige physikalische Prinzip der Flüssigkeitsaufnahme bzw. -abgabe. Entweder erfolgt die Dosierung der Flüssigkeit durch das Luftpolsterprinzip oder es kommt das Prinzip der Direktverdrängung zum Einsatz. Die erste Klasse von Geräten wird als Luftpolsterpipetten bezeichnet, die zweite Klasse als Direktverdränger.

Luftpolsterpipetten verwenden ein Kolben-Zylinder-System, mit dem die eigentliche Abmessung erfolgt. Ein Luftpolster trennt die in eine Kunststoffspitze aufgesaugte Probe vom Kolben im Inneren der Pipette. Beim Heraufgleiten des Kolbens entsteht ein Unterdruck in der Spitze, der das Aufsteigen der Flüssigkeit in die Spitze bewirkt. Das durch den Kolben bewegte Luftpolster wirkt wie eine elastische Feder, an der das Flüssigkeitsvolumen in der Spitze hängt. Bei handgehaltenen Pipettiervorrichtungen, die nach dem Prinzip der Direktverdrängung arbeiten, kommen als Transferbehälter Spitzen mit einem integrierten Kolben zum Einsatz, der mit der Kolbenstange des Dosiergeräts während des Pipettiervorgangs gekoppelt ist und den eigentlichen Dosiervorgang übernimmt. Da der Kolben in diesem Fall das gesamte Innenvolumen der Spitze verdrängen kann, bildet sich zwischen aufgesaugter Probe und Kolbenende im Wesentlichen kein Luftpolster. Beide Klassen von handgehaltenen Pipettiervorrichtungen, die Luftpolsterpipetten und die Direktverdränger, werden jeweils auch unter den Begriff Kolbenhubpipette gefasst.

Es gibt handgehaltene Pipettiervorrichtungen, deren Kolbensystem manuell angetrieben wird, und solche mit elektrischem Antrieb. Eine elektrisch angetriebene, handgehaltene Kolbenhubpipette ist oftmals durch mindestens ein Pipettierprogramm steuerbar, um mindestens eine Art von Pipettiervorgang automatisiert oder teilautomatisiert durchzuführen.

Handgehaltene Pipetten sind für die einhändige Benutzung durch menschliche Nutzer ausgebildet. Es gibt aber auch Laborautomaten mit roboterisierten Greifarmen, deren Greifwerkzeuge die Tätigkeiten einer menschlichen Hand zur Bedienung einer handgehaltenen Pipette nachbilden und die zum Hantieren und Betreiben einer handgehaltenen Pipette eingerichtet sind.

Bei einer Pipettiervorrichtung kann die durch eine einzelne Betätigung abgegebene Probenmenge der in das Gerät aufgesaugten Probenmenge entsprechen. Es kann aber auch vorgesehen sein, dass eine mehreren Abgabemengen entsprechende aufgenommene Probenmenge schrittweise wieder abgegeben wird. Zudem wird zwischen Einkanal-Pipettiervorrichtungen und Mehrkanal-Pipettiervorrichtungen unterschieden, wobei Einkanal-Pipettiervorrichtungen nur einen einzigen Abgabe-/Aufnahmekanal enthalten und Mehrkanal-Pipettiervorrichtungen mehrere Abgabe-/Aufnahmekanäle enthalten, die insbesondere das parallele Abgeben oder Aufnehmen mehrerer Proben erlauben.

Die im Rahmen der vorliegenden Erfindung beschriebenen handgehaltenen Pipettiervorrichtungen sind vorzugsweise, aber nicht ausschließlich, handgehaltene, computergesteuerte Kolbenhubpipetten mit elektrischem Kolbenantrieb, auch bezeichnet als handgehaltene, elektrische Pipettiervorrichtungen oder handgehaltene, elektrische Kolbenhubpipetten.

Ein Beispiel für eine handgehaltene, elektronische Luftpolsterpipette des Stands der Technik ist die Eppendorf Xplorer^{®} und Xplorer^{®} plus der Eppendorf AG, Deutschland, Hamburg; Beispiele für handgehaltene, elektronische Dispenser sind die Multipette^{®} E3 und Multipette^{®} E3x der Eppendorf AG, Deutschland, Hamburg.

Elektrische Pipettiervorrichtungen bieten zahlreiche Vorteile gegenüber nicht-elektrischen Pipettiervorrichtungen, da eine Vielzahl von Funktionen in einfacher Weise implementiert werden kann. Insbesondere lässt sich bei elektrischen Pipettiervorrichtungen die Durchführung von bestimmten, programmgesteuerten Pipettiervorgängen vereinfachen, indem diese automatisiert oder teilautomatisiert werden. Typische Pipettierparameter zum Steuern solcher Pipettiervorgänge mittels entsprechender Pipettierprogramme betreffen das Volumen beim Ansaugen oder Abgeben von Flüssigkeit, deren Reihenfolge und Wiederholungen, und gegebenenfalls deren zeitliche Parameter bei der zeitlichen Verteilung dieser Vorgänge. Eine elektrische Pipettiervorrichtung kann dazu ausgebildet sein, in einem Betriebsmodus oder mehreren Betriebsmodi betrieben zu werden. Ein Betriebsmodus kann vorsehen, dass ein Satz mit einem oder mehreren Pipettierparametern der Pipettiervorrichtung, die einen Pipettiervorgang der Pipettiervorrichtung beeinflussen oder steuern, automatisch abgefragt, eingestellt und/oder angewandt wird.

Es sind handgehaltene Pipettiervorrichtungen bekannt, bei denen ein berührungsempfindlicher Bildschirm verwendet wird, um dem Benutzer die Eingabe von Pipettierparametern zu erlauben, anhand derer ein Pipettiervorgang von der handgehaltenen Pipettiervorrichtung zumindest teilweise automatisiert durchführbar ist.

Ein komfortables Pipettiersystem wird unter der Bezeichnung "VisioNize^{®} pipette manager" bzw. "Connected Pipettes" von der Eppendorf AG, Hamburg, Deutschland vertrieben. Eine produktive und effiziente Arbeitsumgebung wird dabei erzeugt, indem einerseits handgehaltene Pipettiervorrichtungen und zudem ein drahtlos mit diesen kommunizierender portabler PC in einem Geräteverbund agieren. Mittels des portablen PCs sind die Pipettiervorrichtungen konfigurierbar, insbesondere sind Pipettierparameter eines oder mehrerer Pipettiervorgänge festlegbar. Der portable PC ist ein Bedienmodul mit Touchscreen. Im Zentrum der Funktionalität steht eine Software, bezeichnet als "VisioNize^{®} pipette manager", welche die Konfiguration einer oder mehrerer handgehaltener Pipettiervorrichtungen per drahtloser Datenverbindung erlaubt. Bei diesem System lassen sich mehrere Pipettiervorrichtungen desselben Typs oder unterschiedlicher Art ansteuern.

Ein wichtiger Aspekt bei der effizienten Ausübung von Laborarbeit ist die Reproduzierbarkeit von in Experimenten durchgeführten Pipettiermethoden, die eine Abfolge von an Laborproben durchgeführten Pipettiervorgängen beinhalten. Bei dem genannten Pipettiersystem wurde eine Dokumentationsfunktion eingeführt, die der vorliegenden Erfindung zugrunde liegt.

Der vorliegenden Erfindung liegt die Aufgabe zugrunde, ein System zur Aufzeichnung von Pipettiervorgängen in benutzerdefinierten Experimenten bereitzustellen.

Die Erfindung löst diese Aufgabe insbesondere mittels der Gegenstände der unabhängigen Ansprüche. Bevorzugte Ausgestaltungen sind Gegenstände der Unteransprüche und sind zudem der Beschreibung zu entnehmen.

Die Erfindung betrifft ein System zur Aufzeichnung von Pipettiervorgängen in benutzerdefinierten Experimenten, bei denen mindestens eine handgehaltene Pipettiervorrichtung zum Pipettieren mindestens einer Probe verwendet wird. Ein Experiment beinhaltet eine Methode, die durch eine Abfolge von Pipettiervorgängen definiert wird. Ein Parametersatz von Pipettierparametern definiert einen Pipettiervorgang. Ein Pipettiervorgang ist eine Abfolge von Schritten, die von einer Pipettiervorrichtung automatisch bzw. teilautomatisch - oft sind ein oder mehrere Triggereingaben des Benutzers erforderlich- ausgeführt werden und die insbesondere die elektronisch/software-gesteuerte oder manuell gesteuerte Bewegung eines Bewegungselements, insbesondere Kolbens, beinhalten, durch die eine oder mehrere flüssige Laborproben pipettiert werden. "Pipettieren" beinhaltet in diesem Kontext: das Ansaugen mindestens einer flüssigen Probe in mindestens einen Pipettierbehälter, insbesondere eine Pipettenspitze oder Dispenserspritze; ein nachfolgendes Halten der Probe in dem mindestens einen Pipettierbehälter; und die Probenabgabe aus dem mindestens einen Pipettierbehälter in einen oder mehrere Zielbehälter. Der durch die Pipettierparameter definierte Pipettiervorgang kann einem Betriebsmodus der Pipettiervorrichtung zugeordnet sein. Eine Pipettiervorrichtung kann eine Vielzahl von Betriebsmodi anbieten, die den Benutzer bei einer Vielzahl von Anwendungsszenarien unterstützen. Diese werden nachfolgend noch erläutert.

In Laboren durchgeführte Tätigkeiten, bei denen Pipettiervorgänge eingesetzt werden, folgen einem Ablauf, der letztlich von jedem Benutzer individuell vorgebbar oder zumindest beeinflussbar ist. Die Summe der Tätigkeiten eines Benutzers, die dieser vornimmt, um eine oder mehrere flüssige Proben ausgehend von einem Anfangszustand bis zu einem Endzustand zu bearbeiten, wird als Experiment bezeichnet. Typischerweise werden nicht nur eine Anzahl von Proben verwendet, sondern auch eine Vielzahl von unterschiedlichen Pipettiervorrichtungen und dazu kompatiblen Pipettierbehältern, die insbesondere unterschiedliche Nennvolumina aufweisen können. In einem Experiment wird die Effizienz durch eine Protokollierfunktion erheblich verbessert, wenn die einzelnen Pipettiervorgänge nicht manuell vom Benutzer in einem Laborbuch aufgezeichnet werden müssen sondern elektronisch/softwaregesteuert erfasst werden.

Typisch für elektrische Pipettiervorrichtungen ist, dass vom Benutzer die erforderlichen Pipettierparameter vor Durchführung festgelegt werden. Zu diesem Zweck ist eine Datenverarbeitungseinrichtung dazu programmiert, mittels einer Benutzerschnittstelleneinrichtung, z.B. eines Touchscreens, Parameterwerte benutzerdefinierbarer Pipettierparameter mindestens eines Parametersatzes zu erfassen, die den mindestens einen, mittels der mindestens einen handgehaltenen Pipettiervorrichtung durchzuführenden, Pipettiervorgang definieren.

Eine Pipettiervorrichtung weist vorzugsweise mindestens ein Betätigungselement auf, mit dem der Benutzer Eingaben an der Pipettiervorrichtung vornehmen kann, insbesondere einen dann automatisch durchgeführten Pipettiervorgang -und/oder dessen Teilvorgängestarten kann. Die Betätigungen des mindestens einen Betätigungselements im Verlauf eines Pipettiervorgangs und/oder die Summe der Ereignisse im Verlauf eines oder mehrerer Pipettiervorgänge einer Methode können durch die Datenverarbeitungseinrichtung ebenso in Aufzeichnungsdaten aufgezeichnet werden wie, vorzugsweise jeweils optional, Informationen über mindestens eines der folgenden: einen verwendeten Betriebsmodus, die beim Pipettiervorgang angewandten Pipettierparameter, Anfangs- und/oder Endzeit der Pipettiervorgänge oder deren Teilschritte, Identifikationsdaten -z.B. Seriennummern- der eingesetzten individuellen Pipettiervorrichtungen, Identifikationsdaten des -gegebenenfalls zuvor authentifizierten oder ausgewählten- Benutzers, der in der Regel der ausführende Verantwortliche eines Experiments sein wird, sowie andere Daten, die von anderen am Experiment beteiligten Geräten und/oder Sensoren eines Labors bereitgestellt sein können.

Die Dokumentierfunktion wird im System implementiert, indem als Systemmerkmal eine Methodenaufzeichnung vorgesehen ist, die mittels einer entsprechend programmierten Datenverarbeitungseinrichtung umgesetzt ist. Wenn diese Methodenaufzeichnung als "erste Methodenaufzeichnung" bezeichnet wird, dient dies lediglich einer Namensgebung, die den Rückbezug auf dieses Systemmerkmal erlaubt. Systemintern ist die erste Methodenaufzeichnung über "erste Methoden-Identifikationsdaten" referenzierbar. Die Begriffe "erste" und "zweite", etwa auch "dritte" usw., die im Rahmen dieser Beschreibung zur Bezeichnung von Gegenständen verwendet werden, sollen typischerweise als Namensgebung verstanden werden, und dienen in der Regel nicht zur Bezeichnung einer Reihenfolge oder Benennung einer Anzahl, es sei denn dies ergibt sich im jeweiligen Kontext als zutreffend. Insbesondere bedeutet die Nennung "erste Methodenaufzeichnung" und "zweite Methodenaufzeichnung" nicht, dass keine dritte und weitere Methodenaufzeichnung möglich wäre, die Bezeichnung "erste" und "zweite" dienen in diesem Kontext vor allem der Unterscheidung der Methodenaufzeichnungen.

Die Erfindung sieht ausdrücklich vor, dass auch eine Mehrzahl von Methodenaufzeichnungen parallel zueinander durchgeführt werden können. Die Datenverarbeitungseinrichtung kann programmiert sein, Parameterwerte von mehreren im Wesentlichen parallel stattfindenden Pipettiervorgängen zu verarbeiten und insbesondere in derselben Methodenaufzeichnung oder in unterschiedlichen Methodenaufzeichnungen zu speichern. Beispielsweise könnte, in einem Ausnahmefall, ein Benutzer jeweils mit einer Hand eine bestimmte Pipettiervorrichtung zeitgleich bedienen, und deren Parameterwerte könnten im Wesentlichen parallel zur ersten Kommunikationseinrichtung übertragen werden, um in derselben Methodenaufzeichnung oder in unterschiedlichen Methodenaufzeichnungen gespeichert zu werden. Diese Art der parallelen Aufzeichnung ist nicht zu verwechseln mit der verschachtelten Aufzeichnung, die durch das erfindungsgemäße System ermöglicht wird.

Die Datenverarbeitungseinrichtung ist vorzugsweise dazu programmiert, mindestens eine Pipettiervorrichtung zur Verwendung mit der ersten Methode zu registrieren, indem insbesondere die Identifikationsdaten, insbesondere eine Seriennummer der Pipettiervorrichtung, insbesondere gemeinsam mit den ersten Methoden-Identifikationsdaten im ersten Methodendatensatz, gespeichert werden.

Die Datenverarbeitungseinrichtung ist vorzugsweise dazu programmiert, bei der Registrierung einer Pipettiervorrichtung zur Verwendung mit der ersten Methode anhand der Identifikationsdaten der Pipettiervorrichtung zu prüfen, ob die Pipettiervorrichtung bereits in einer anderen laufenden -also gestarteten und noch nicht beendeten, Methodenaufzeichnung registriert ist, und insbesondere, falls dies der Fall ist, die erste Pipettiervorrichtung nicht zur Verwendung mit der ersten Methode zu registrieren. Auf diese Weise wird eine Pipettiervorrichtung nur genau einer laufenden Methodenaufzeichnung zugeordnet sein, was Verwechslungen seitens des Nutzers verhindert und die Sicherheit der Arbeit mit dem System verbessert.

Das Pausieren einer Methodenaufzeichnung erlaubt einerseits ein undokumentiertes Arbeiten, also Durchführen von Pipettiervorgängen mit mindestens einer Pipettiervorrichtung, die -zumindest vor der Aufzeichnungspause- zur Verwendung mit der ersten Methode registriert ist bzw. war. Andererseits lässt sich diese mindestens eine Pipettiervorrichtung einer anderen, während der Aufzeichnungspause gestarteten oder laufenden, Methodenaufzeichnung per Registrierung zuordnen, was eine effizientere Nutzung vorhandener Pipettiervorrichtungen unter im Wesentlichen kontinuierlicher Nutzung einer Dokumentationsfunktion ermöglicht. Diese Art der Verschachtelung von Methodenaufzeichnungen ist deshalb eine bevorzugte Weiterbildung der Erfindung.

Während der ersten Methodenaufzeichnung wird in der Datenspeichereinrichtung insbesondere eine erste Abfolge von Parametersätzen aufgezeichnet, welche einer mittels der mindestens einen handgehaltenen Pipettiervorrichtung durchgeführten ersten Abfolge von Pipettiervorgängen entspricht, die bei einem ersten Experiment verwendet werden. In einem ersten Aufzeichnungsabschnitt der ersten Methodenaufzeichnung wird insbesondere eine erste Untermenge der ersten Abfolge von Pipettiervorgängen im ersten Methodendatensatz gespeichert. In einem zweiten Aufzeichnungsabschnitt der ersten Methodenaufzeichnung wird insbesondere eine zweite Untermenge der ersten Abfolge von Pipettiervorgängen im ersten Methodendatensatz gespeichert. In einem dritten (i-ten, i eine natürliche Zahl) Aufzeichnungsabschnitt der ersten Methodenaufzeichnung wird insbesondere eine dritte (i-te) Untermenge der ersten Abfolge von Pipettiervorgängen im ersten Methodendatensatz gespeichert. Zwischen den Aufzeichnungsabschnitten liegt insbesondere jeweils eine Aufzeichnungspause. Die Pipettiervorgänge einer Methodenaufzeichnung werden also, insbesondere durch eine oder mehrere Pausen, unterbrochen, sukzessive im ersten Methodendatensatz gesammelt und gespeichert. Die Pausendauer ist zeitlich nicht beschränkt und hängt vom Benutzervorgehen ab.

Das erfindungsgemäße System bzw. die Dokumentierfunktion ist vorzugsweise dadurch gekennzeichnet, dass die Methodenaufzeichnung pausiert werden kann. Damit ist nicht ausschließlich gemeint, dass zwischen den Zeitintervallen der Aufzeichnung von Aufzeichnungsabschnitten einer Methodenaufzeichnung ein zeitlicher Versatz existiert. Vielmehr ist vorzugsweise vorgesehen, dass ein Benutzer die Methodenaufzeichnung durch manuelle Eingabe am System, insbesondere an der Benutzerschnittstelleneinrichtung, aktiv pausiert, nämlich in Abhängigkeit von einer Benutzereingabe am System, insbesondere an der Benutzerschnittstelleneinrichtung. Dadurch kann der Benutzer insbesondere einen oder mehrere Pipettiervorgänge durchführen oder sonstige Ereignisse -z.B. Konfigurationen- können an den Pipettiervorrichtungen des Systems, bzw. an den zur Verwendung mit einer Methode registrierten Pipettiervorrichtungen ausgeführt werden, die dann nicht in der Methodenaufzeichnung aufgezeichnet werden. Nach der Pause kann wahlweise die Methodenaufzeichnung fortgesetzt oder auch beendet werden. Die Datenverarbeitungseinrichtung ist vorzugsweise dazu programmiert, dass im Status der Pausierung -für den Benutzer wahlweise- ein Fortsetzen der ersten Methodenaufzeichnung oder deren Beenden möglich ist.

Die Pausierung (auch bezeichnet als: das Anhalten) ist nicht gleichbedeutend mit dem Beenden der Methodenaufzeichnung. Die Pausierungsoption verleiht dem System eine zuvor nicht bekannte Flexibilität bei der Durchführung von Experimenten und deren Protokollierung in Laboren. Die Datenverarbeitungseinrichtung ist vorzugsweise dazu programmiert, dass einer Methodenaufzeichnung, die durch Beendigung den Status "beendet" erhalten hat, keine weiteren Aufzeichnungsdaten, insbesondere keine Parameterwerte, durch Speichern im ersten Methodendatensatz hinzugefügt werden können. Beispielsweise kann dem ersten Methodendatensatz aber nach Beendigung der ersten Methodenaufzeichnung ein Verifikationswert zugeordnet werden, um eine unentdeckte Manipulation des ersten Methodendatensatzes nach Beendigung der ersten Methodenaufzeichnung auszuschließen.

Die Datenverarbeitungseinrichtung ist vorzugsweise dazu programmiert:
a) in Abhängigkeit von einer Benutzereingabe die Aufzeichnung eines ersten Aufzeichnungsabschnitts der ersten Methodenaufzeichnung zu starten, bei dem eine erste Untermenge der ersten Abfolge von Parametersätzen des ersten Experimentabschnitts aufgezeichnet und als erster Methodendatensatz in der Datenspeichereinrichtung gespeichert wird,
b) in Abhängigkeit von einer Benutzereingabe die erste Methodenaufzeichnung zu pausieren.

Vorzugsweise ist dann vorgesehen: c) in Abhängigkeit von einer Benutzereingabe und in Abhängigkeit von den ersten Methoden-Identifikationsdaten den gespeicherten ersten Methodendatensatz auszuwählen und die das erste Experiment aufzeichnende erste Methodenaufzeichnung zu beenden oder fortzusetzen, indem im Falle der Fortsetzung die Aufzeichnung eines zweiten Aufzeichnungsabschnitts der ersten Methodenaufzeichnung gestartet wird, bei dem die zweite Untermenge der ersten Abfolge von Parametersätzen des zweiten Experimentabschnitts aufgezeichnet wird. Im Falle des Beendens der ersten Methodenaufzeichnung ist die Datenverarbeitungseinrichtung vorzugsweise dazu programmiert, dass eine weitere Fortsetzung der ersten Methodenaufzeichnung und/oder insbesondere deren nachträgliche Änderung nicht möglich ist.

Durch die Fortsetzbarkeit der ersten Methodenaufzeichnung ergeben sich zahlreiche vorteilhafte Anwendungsszenarien der Protokollierung: Es können eine Anzahl N>1 an Methoden verschachtelt, parallel ausgeführt und auch aufgezeichnet werden. Dazu kann die erste Methodenaufzeichnung pausiert werden, eine zweite Methodenaufzeichnung fortgesetzt oder gestartet werden, die zweite Methodenaufzeichnung pausiert werden, eine dritte Methodenaufzeichnung fortgesetzt oder gestartet werden, die dritte Methodenaufzeichnung pausiert werden usw.

Ein Verfahren zur Verwendung des Systems kann insbesondere folgende sukzessive Arbeitsweise vorsehen, die insbesondere folgende sukzessiv auszuführenden Schritte beinhaltet, die Bestandteile eines Aufzeichnungsabschnitts sein können:
a1) Konfigurieren mindestens einer Pipettiervorrichtung durch mindestens einen ersten Parametersatz, der vom Benutzer an der Benutzerschnittstelleneinrichtung vorgegeben wird;
a2) Durchführen mindestens eines Pipettiervorgangs gemäß der Konfiguration der mindestens einen Pipettiervorrichtung durch den mindestens einen ersten Parametersatz;
a3) Aufzeichnen der Parameterwerte des ersten Parametersatzes im ersten Methodendatensatz;
a4) Konfigurieren mindestens einer Pipettiervorrichtung durch mindestens einen zweiten Parametersatz, der vom Benutzer an der Benutzerschnittstelleneinrichtung vorgegeben wird;
a5) Durchführen mindestens eines Pipettiervorgangs gemäß der Konfiguration der mindestens einen Pipettiervorrichtung durch den mindestens einen zweiten Parametersatz;
a6) Aufzeichnen der Parameterwerte des ersten Parametersatzes im ersten Methodendatensatz;
insbesondere kann entsprechend allgemein die folgende Sequenz mehrfach bzw. beliebig oft -und für jede beliebige Methodenaufzeichnung- wiederholt werden (j=1... n; n eine natürliche Zahl):
a_i) Konfigurieren mindestens einer Pipettiervorrichtung durch mindestens einen j-ten Parametersatz, der vom Benutzer an der Benutzerschnittstelleneinrichtung vorgegeben wird;
a_ii) Durchführen mindestens eines Pipettiervorgangs gemäß der Konfiguration der mindestens einen Pipettiervorrichtung durch den mindestens einen j-ten Parametersatz
a_iii) Aufzeichnen der Parameterwerte des j-ten Parametersatzes im ersten Methodendatensatz.

Alternativ zu einer sukzessiven Konfiguration der Pipettiervorgänge könnte die Datenverarbeitungseinrichtung dazu programmiert sein, eine vom Benutzer an der Benutzerschnittstelleneinrichtung definierte Sequenz von Parametersätzen zu ermitteln, mittels derer mindestens eine Pipettiervorrichtung für mehrere sukzessive Pipettiervorgänge konfiguriert wird, wobei die entsprechenden Aufzeichnungsdaten sukzessive -z.B. nach Beendigung eines jeweiligen, durch einen Parametersatz definierten Pipettiervorgangs- an die erste Kommunikationseinrichtung zum Zwecke der Aufzeichnung übertragen werden können, oder wobei die Aufzeichnungsdaten nicht sukzessive, sondern kontinuierlich, beispielsweise am Ende einer Methode oder eines Aufzeichnungsabschnitts, an die erste Kommunikationseinrichtung zum Zwecke der Aufzeichnung übertragen werden können.

Die Methodenaufzeichnung(en) kann/können ein und demselben Benutzer zugeordnet sein oder unterschiedlichen Benutzern. Da die Methodenaufzeichnungen zeitlich verschachtelt sind, ermöglicht dies die Nutzung ein und derselben Pipettiervorrichtung -z-B. durch deren Identifikationsdaten identifiziert- in mehreren der zeitlich verschachtelten Methodenaufzeichnungen.

In einem typischen Anwendungsszenario führt ein Benutzer in einem ersten Experiment an ersten Proben eine erste Methode durch -z.B. Aufteilen einer Stammlösung -z.B. einer Zellsuspension- auf Einzelbehälter, Verteilen der Teile auf Einzelröhrchen, Hinzupipettieren von einzumischender Flüssigkeit in jedes Einzelröhrchen. Dann kann das Zentrifugieren der Proben in den Einzelröhrchen erforderlich sein. Dabei entsteht eine Wartezeit innerhalb des ersten Experiments. Durch Pausieren der ersten Methodenaufzeichnung kann der Benutzer ein zweites, ebenfalls aufgezeichnetes Experiment beginnen, insbesondere unter Verwendung derselben Pipettiervorrichtungen, die bereits bei der ersten Methode verwendet wurden und dieser, insbesondere per Registrierung, zugeordnet sind, ohne eine unproduktive Arbeitspause einlegen zu müssen. Im Prinzip ist die Anzahl N der verschachtelt aufzeichenbaren Methoden nicht begrenzt, insbesondere N>5, N>10 und insbesondere N zwischen 1 und 100.

Die Datenverarbeitungseinrichtung ist vorzugsweise dazu programmiert, ein Authentifizierungsverfahren durchzuführen, bei dem ein Benutzer in einer Sitzung (session) angemeldet wird und in Abhängigkeit von diesem Benutzer Benutzeridentifikationsdaten bestimmt werden, insbesondere durch Auswahl aus einer Sammlung bestehender Benutzeridentifikationsdaten oder durch Zuordnung neu bestimmter, individueller Benutzeridentifikationsdaten. Die Methodenaufzeichnung erfolgt insbesondere in Abhängigkeit von Benutzeridentifikationsdaten, insbesondere werden Benutzeridentifikationsdaten mit der Methodenaufzeichnung in der Datenspeichereinrichtung gespeichert.

Die Datenverarbeitungseinrichtung ist vorzugsweise dazu programmiert, Nutzer des Systems zu unterscheiden, insbesondere durch ein Authentifizierungsverfahren. Dieses Vorgehen wird als Bestandteil eines optionalen, insbesondere von einem Nutzer aktivierbaren und/oder deaktivierbaren, Nutzermanagement angesehen, wobei dieser Nutzer nutzergruppenspezifische Rechte haben kann; insbesondere kann dieser Nutzer ein Administrator-Nutzer sein, der gegenüber einem Standardnutzer das Recht zum Aktivieren und/oder Deaktivieren des Nutzermanagements haben kann. Wenn das Nutzermanagement aktiviert ist, kann nur der jeweilige Nutzer auf eine ihm zugeordnete Methode zugreifen und diese fortsetzen. Wenn das Nutzermanagement deaktiviert ist, können verschiedene Nutzer auf eine Methode zugreifen (und diese damit auch fortsetzen). Bsp.: Labormitarbeiter 1 startet Methode am Vormittag, Labormitarbeiter 2 führt die Methode am Nachmitttag fort.

Eine handgehaltene Pipettiervorrichtung zum Pipettieren mindestens einer flüssigen Probe kann eine nach dem Luftpolsterprinzip arbeitende Kolbenhubpipette sein oder eine nach dem Direktverdrängerprinzip arbeitende Repettierpipette, auch als Dispenser bezeichnet. Die handgehaltene Pipettiervorrichtung weist insbesondere auf:
* einen Verbindungsabschnitt zum Verbinden mindestens eines Pipettierbehälters ;
* ein elektrisch gesteuertes Bewegungselement zum Ansaugen mindestens einer Probe in mit den mindestens einen Pipettierbehälter, Halten der Probe in dem mindestens einen Pipettierbehälter und Probenabgabe aus dem mindestens einen Pipettierbehälter bei der Durchführung eines Pipettiervorgangs; und
* eine elektrische Steuereinrichtung, die zur Steuerung des Bewegungselements in Abhängigkeit von dem mindestens einen Pipettierparameter eingerichtet ist;
Vorzugsweise: * eine Kommunikationseinrichtung zum Empfangen der Parameterwerte der benutzerdefinierbaren Pipettierparameter von einem entfernt von der Pipettiervorrichtung angeordneten Computergerät, insbesondere einem portablen PC, insbesondere einem Tablet-PC.

Das, insbesondere portable, Computergerät weist, jeweils vorzugsweise auf:
- diese Benutzerschnittstelleneinrichtung mit dem Display,
- diese Datenverarbeitungseinrichtung,
- optional : die erste Kommunikationseinrichtung zum Senden der Parameterwerte der benutzerdefinierbaren Pipettierparameter an die mindestens eine zweite Kommunikationseinrichtung der mindestens einen handgehaltenen Pipettiervorrichtung, und
- optional: diese Datenspeichereinrichtung,
- ein Gehäuse, in und/oder an dem die genannten Bestandteile angeordnet sind, und das insbesondere von einem Benutzer einhändig gehalten und das Computergerät insbesondere einhändig bedient werden kann.

Durch die Portabilität der Komponenten ist das System besonders flexibel und einfach im Labor verwendbar.

Anstelle eines portablen Computergeräts kann auch ein nicht-portables Computergerät, z.B. ein stationärer PC, bei ansonsten gleicher Funktionalität verwendet werden. Die Benutzerschnittstelleneinrichtung mit dem Display kann prinzipiell auch Bestandteil einer Pipettiervorrichtung sein.

Vorzugsweise ist die Datenverarbeitungseinrichtung dazu programmiert, um, nach dem Pausieren der ersten Methodenaufzeichnung in Schritt b), mittels der Benutzerschnittstelleneinrichtung mindestens einen weiteren Parametersatz mindestens eines weiteren Pipettiervorgangs zu erfassen, der keinen Bestandteil der ersten Abfolge von Pipettiervorgängen bildet, und wobei insbesondere die Steuereinrichtung dazu eingerichtet ist, nach dem Pausieren der ersten Methodenaufzeichnung in Schritt b), das Bewegungselement in Abhängigkeit von dem mindestens einen weiteren Parametersatz zu steuern. Durch diesen Schritt wird der Vorteil umgesetzt, dass ein Pausieren der Methodenaufzeichnung auch das Pausieren eines protokollierten Experiments ermöglicht, wobei während der Pause in Form des Weiteren Pipettiervorgangs produktiv weitergearbeitet werden kann, der zumindest vom Benutzer konfiguriert oder auch vom Benutzer tatsächlich ausgeführt werden kann.

Vorzugsweise ist die Datenverarbeitungseinrichtung dazu programmiert, um nach dem Pausieren der ersten Methodenaufzeichnung in Schritt b) und vor dem Start der Aufzeichnung des zweiten Aufzeichnungsabschnitts der ersten Methodenaufzeichnung in Schritt c), eine zweite Methodenaufzeichnung durchzuführen, der zweite (von den ersten Methoden-Identifikationsdaten verschiedene) Methoden-Identifikationsdaten zugeordnet sind und die in der Datenspeichereinrichtung eine zweite Abfolge von Parametersätzen aufzeichnet, welche einer mittels der mindestens einen handgehaltenen Pipettiervorrichtung durchgeführten zweiten Abfolge von Pipettiervorgängen entspricht, die bei einem zweiten Experiment verwendet werden, insbesondere auch Zeitdaten dieser Pipettiervorgänge aufzeichnet. Durch diesen Schritt wird der Vorteil umgesetzt, dass in einer Pause des ersten Experiments ein zweites Experiment begonnen und auch aufgezeichnet werden kann, ohne die Aufzeichnung des ersten Experiments zu stören, die später fortgesetzt oder beendet werden kann. Ähnlich zu dem Fall, dass ein zweites Experiment (eine zweite Methode) nach Schritt b) begonnen wird, kann auch ein zweites Experiment fortgesetzt werden, vorausgesetzt, dass es bereits begonnen wurde, dessen Aufzeichnung bereits gestartet und zwischenzeitlich pausiert wurde. Die Anzahl der verschachtelten Experimente bzw. Methodenaufzeichnungen ist, wie bereits betont, nicht auf eine "erste" und eine "zweite" Methodenaufzeichnung beschränkt, es kann eine beliebige Anzahl von Methoden/Experimenten verschachtelt aufgezeichnet werden.

Vorzugsweise ist die Datenverarbeitungseinrichtung dazu programmiert, auf dem Display der Benutzerschnittstelleneinrichtung
* eine Bildschirmseite mit einer Schaltfläche anzuzeigen, deren Betätigen bzw. Berühren eine Methodenaufzeichnung startet.
   Vorzugsweise ist die Datenverarbeitungseinrichtung dazu programmiert, auf dem Display der Benutzerschnittstelleneinrichtung
* mindestens eine Bildschirmseite anzuzeigen, in welcher der Titel mindestens einer laufenden Methodenaufzeichnung angezeigt wird, insbesondere in welcher die Titel mehrerer laufender Methodenaufzeichnungen aufgelistet sind,
* mindestens ein Informationsfeld anzuzeigen, das den Status der entsprechenden Methodenaufzeichnung anzeigt, der beinhalten kann: Aufzeichnung ist pausiert oder Aufzeichnung läuft oder Aufzeichnung ist beendet.

Vorzugsweise ist die Datenverarbeitungseinrichtung dazu programmiert, auf dem Display der Benutzerschnittstelleneinrichtung
* eine Bildschirmseite anzuzeigen, in welcher der Titel mindestens einer laufenden Methodenaufzeichnung angezeigt wird, insbesondere in welcher die Titel mehrerer laufender Methodenaufzeichnungen aufgelistet sind,
* mindestens eine Schaltfläche anzuzeigen, deren Betätigen bzw. Berühren die mindestens eine, mit der Schaltfläche assoziierte, zuvor pausierte Methodenaufzeichnung auswählt;
* und eine Schaltfläche anzuzeigen, deren Betätigen bzw. Berühren die ausgewählte pausierte Methodenaufzeichnung fortsetzt, insbesondere während mindestens eine andere Methodenaufzeichnung pausiert ist.

Vorzugsweise ist die Datenverarbeitungseinrichtung dazu programmiert, auf dem Display der Benutzerschnittstelleneinrichtung
* eine Bildschirmseite anzuzeigen, in welcher der Titel mindestens einer laufenden Methodenaufzeichnung angezeigt wird, insbesondere in welcher die Titel mehrerer laufender Methodenaufzeichnungen aufgelistet sind,
* mindestens eine Schaltfläche anzuzeigen, deren Betätigen bzw. Berühren die mindestens eine, mit der Schaltfläche assoziierte, zuvor pausierte Methodenaufzeichnung auswählt;
* und eine Schaltfläche anzuzeigen, deren Betätigen bzw. Berühren die ausgewählte pausierte Methodenaufzeichnung fortsetzt.

Vorzugsweise ist die Datenverarbeitungseinrichtung dazu programmiert, mindestens einen Methodendatensatz mindestens einer beendeten Methodenaufzeichnung als Datei bereitzustellen, die auf ein externes Datenverarbeitungsgerät übertragbar ist, oder deren Inhalt ausgebbar ist durch Drucken, Anzeige auf dem Bildschirm, oder Übertragen zu einem externen Datenverarbeitungsgerät des Systems oder einem Server, der nicht Bestandteil des Systems ist.

Wenn von automatisierten Pipettiervorgängen gesprochen wird, sind stets auch teilautomatisierte Pipettiervorgänge gemeint, es sei denn, der jeweilige Kontext widerspricht dem. Zum Durchführen eines automatisierten Pipettiervorgangs gehört immer auch mindestens eine Benutzertätigkeit, die zumindest den automatisierten Pipettiervorgang auslöst, in der Regel durch das Betätigen eines Bedienelements der Pipettiervorrichtung. Teilautomatisierte Pipettiervorgänge bestimmter Anwendungsszenarien können beispielsweise erfordern, dass ein Benutzer, zusätzlich zum Starten eines in Teilvorgänge gegliederten Pipettiervorgangs, zumindest auch das Auslösen eines Teilvorgangs durch Betätigung eines Bedienelements startet. Die automatisierten Pipettiervorgänge eines bestimmten Anwendungsszenarios können bei bestimmten Ausführungsformen der Pipettiervorrichtung durch einen Satz von Pipettierparametern definiert sein, die der Benutzer vor dem Start eines automatisierten Pipettiervorgangs mittels der Bedieneinrichtung einer Benutzerschnittstelleneinrichtung der Pipettiervorrichtung einstellt, und/oder aus Default-Einstellungen übernimmt und/oder aus einem Speicher lädt. Typischerweise weist die Benutzerschnittstelle von solchen Ausführungsformen der Pipettiervorrichtung eine Auswahloption auf, zum Beispiel ein Bedienrad oder eine Liste von auswählbaren Feldern auf einem Touchscreen, mittels dem ein so genannter "Betriebsmodus" auswählbar ist, der das spezifische Anwendungsszenario repräsentiert bzw. in dem der ganze Satz von Pipettierparametern ausgewählt ist, die zu diesem spezifischen Anwendungsszenario gehören und die der Benutzer vor dem Start des zugeordneten automatisierten Pipettiervorgangs einstellen kann. Der automatisierte Pipettiervorgang läuft dann gemäß einem als Betriebsprogramm vorgegebenen Pipettierprogramm dieses Betriebsmodus ab.

In einer bevorzugten Ausführungsform ist das System, insbesondere die Pipettiervorrichtung bzw. Datenverarbeitungseinrichtung, dazu programmiert, dass der Benutzer einen automatisierten Pipettiervorgang durch Auswählen von Pipettierparametern manuell definiert, und so insbesondere einen Satz von Pipettierparametern (Pipettierparametersatz) eines benutzerdefinierten Pipettiervorgangs definiert. Dieser Pipettierparametersatz kann einem vorgegebenen Pipettierparametersatz eines Betriebsmodus teilweise oder vollständig entsprechen. Pipettierparameter eines Pipettiervorgangs betreffen bzw. quantifizieren vorzugsweise das zu pipettierende Volumen beim Schritt des Ansaugens der Probe in einen mit der Kolbenhubpipette verbundenen Pipettierbehälter oder beim Schritt des Abgebens der Probe aus diesem Pipettierbehälter, gegebenenfalls die Reihenfolge und Wiederholungen dieser Schritte, und gegebenenfalls zeitliche Parameter bei der zeitlichen Verteilung dieser Vorgänge, insbesondere auch die zeitliche Veränderung solcher Vorgänge, insbesondere die Geschwindigkeit und/oder Beschleunigung des Ansaugens oder Abgebens der Probe. Der automatisierte Pipettiervorgang läuft in dieser Ausführungsform gemäß eines Pipettierprogramms ab, das gemäß diesen benutzerdefinierten Pipettierparametern vollständig definiert ist.

Die erfindungsgemäße handgehaltene Pipettiervorrichtung ist vorzugsweise dazu ausgebildet, zur Durchführung mindestens eines Pipettiervorgangs gemäß mindestens einem Parametersatz von mindestens einem, oder mindestens zwei, oder mindestens drei Pipettierparametern verwendet zu werden, insbesondere in mindestens einem vorbestimmten Betriebsmodus gemäß einem vorbestimmten Parametersatz der Pipettiervorrichtung oder in einem benutzerdefinierten Parametersatz verwendet zu werden. In einem Betriebsmodus ist vorzugsweise jeweils ein Parametersatz von Pipettierparametern (Pipettierparametersatz) vorgesehen, dessen Pipettierparameter optional vom Benutzer nicht auswählbar sind oder optional vom Benutzer zumindest teilweise abwählbar und um weitere Pipettierparameter ergänzbar sind.

Ein Pipettiervorgang sieht typischer Weise vor, dass gemäß einem Pipettierprogramm eine bestimmte Probenmenge aus einem Startbehälter in einen mit der Kolbenhubpipette verbundenen Pipettierbehälter, insbesondere Pipettenspitze, aufgenommen wird und/oder in einen Zielbehälter abgegeben wird, insbesondere dosiert abgegeben wird. Ein Pipettiervorgang lässt sich vorzugsweise durch mindestens einen, zwei, drei oder vorzugsweise mehrere Pipettierparameter steuern, mit dem der genannte Pipettiervorgang, oder eine Funktion bzw. Bestandteil desselben in der gewünschten Weise beeinflussbar oder definierbar ist.

Pipettierparameter zum Steuern eines Pipettiervorgangs betreffen bzw. quantifizieren vorzugsweise das zu pipettierende Volumen, beim Schritt des Ansaugens der Probe in einen mit der Kolbenhubpipette verbundenen Pipettierbehälter oder beim Schritt des Abgebens der Probe aus diesem Pipettierbehälter, gegebenenfalls die Reihenfolge und Wiederholungen dieser Schritte, und gegebenenfalls zeitliche Parameter bei der zeitlichen Verteilung dieser Vorgänge, insbesondere auch die zeitliche Veränderung solcher Vorgänge, insbesondere die Geschwindigkeit und/oder Beschleunigung des Ansaugens oder Abgebens der Probe, oder die Anzahl von Vorbenetzungsschritten und optional das dabei jeweils angesaugte und abgegebene Volumen.

Diese Pipettierparameter werden vorzugsweise zumindest teilweise und vorzugsweise vollständig vom Benutzer ausgewählt und/oder eingegeben, insbesondere über das mindestens eine Bedienelement der Benutzerschnittstelleneinrichtung einer Kolbenhubpipette oder eines externen Datenverarbeitungsgeräts.

Der Pipettiervorgang ist vorzugsweise durch den Pipettierparametersatz eindeutig festlegbar bzw. festgelegt. Dieser Pipettierparametersatz wird vorzugsweise zumindest teilweise und vorzugsweise vollständig vom Benutzer ausgewählt und/oder eingegeben, insbesondere über die Bedieneinrichtung der Pipettiervorrichtung oder des externen Datenverarbeitungsgeräts.

Es ist jedoch möglich, dass ein Pipettiervorgang durch den Pipettierparametersatz nicht eindeutig festgelegt ist; in diesem Fall ist die Pipettiervorrichtung insbesondere dazu eingerichtet bzw. die Datenverarbeitungseinrichtung dazu programmiert, den Pipettierparametersatz durch mindestens einen weiteren Pipettierparameter automatisch zu ergänzen, insbesondere in Abhängigkeit von den (gewählten) Pipettierparametern. Es ist möglich und bevorzugt, dass mindestens ein Pipettierparameter nicht vom Benutzer festgelegt wird, sondern z.B. von der Pipettiervorrichtung vorgegeben wird, indem er dort z.B. vorbekannt in einem Datenspeicher gespeichert ist oder automatisch ermittelt wird.

Vorzugsweise ist mindestens ein Pipettierparameter vorgesehen, mit dem die Zahl von unmittelbar aufeinander folgenden oder mittelbar aufeinander folgenden Pipettiervolumina festgelegt wird, vorzugsweise mindestens ein Pipettierparameter, mit dem die Zahl der Ansaugschritte und/oder der Abgabeschritte und jeweils vorzugsweise auch die jeweils zugehörigen Pipettiervolumina, die jeweils zugehörigen Pipettiergeschwindigkeiten und/oder -beschleunigungen, und/oder die jeweils zugehörigen Zeitabstände zwischen den Schritten festgelegt wird.

Typische Pipettierparameter, die zur manuellen Definition von benutzerdefinierten Pipettiervorgängen herangezogen werden können, lassen sich aus der nachfolgenden Beschreibung typischer Anwendungsszenarien von automatisierten Pipettiervorgängen entnehmen. Jedes Anwendungsszenario kann durch einen entsprechend bezeichneten Pipettiermodus voreingestellt sein. Einem Pipettiermodus kann eine Pipettiermodus-ID zugeordnet sein. Die Pipettierparameter eines Pipettiermodus können an der Benutzerschnittstelleneinrichtung- einer entsprechend gestalteten GUI, siehe Figuren- vom Benutzer eingestellt werden, die Pipettiervorrichtung kann die Pipettierparameter optional gemeinsam mit der Pipettiermodus-ID empfangen und den Pipettiermodus dementsprechend an der Pipettiervorrichtung einstellen.

Ein typisches Anwendungsszenario, das bei Pipettiervorrichtungen als teilautomatisierter Vorgang vorgesehen ist, ist das Dispensieren (kurz bezeichnet als "DIS") eines in den Pipettierbehälter (Pipettenspitze oder Dispenserspitze) aufgenommenen Volumens. Jede Teilabgabe, z.B. das Dispensieren einer Gesamtprobe von 1 ml aus einem Pipettierbehälter in insgesamt zehn getrennte Zielbehälter einer Mikrotiterplatte erfordert es, dass der Benutzer die Pipettiervorrichtung oberhalb eines Vorratsbehälters mit zu verteilender Probenflüssigkeit positioniert, die Spitze des Pipettierbehälters in die Vorratsflüssigkeit eintaucht, durch Betätigung eines Bedienelements ein Startvolumen aufnimmt, durch Betätigung eines Bedienelements oder automatisiert einen Umkehrhub auslöst, wodurch das System in eine definierte Ausgangsposition gebracht wird, oberhalb des ersten Zielbehälters positioniert, die elektrisch getriebene Abgabe eines dosierten Teilvolumens von 0,1 ml durch Betätigung eines Bedienelements startet, die Pipettiervorrichtung oberhalb des nächsten Zielbehälters der Mikrotiterplatte bewegt und positioniert, erneut die elektrisch getriebene Abgabe eines dosierten Teilvolumens von 0,1 ml durch Betätigung eines Bedienelements startet, und diese manuell durchgeführten Teilvorgänge (Positionieren, Auslösen), gefolgt vom automatisierten Teilvorgang der elektrisch getriebenen Teilabgabe von 0,1 ml Probe (von insgesamt 10 Schritten der Teilabgabe) noch acht weitere Male wiederholt. Die dosierte Abgabe der Teilmengen erfolgt nach Vorgabe von Pipettierparametern beinhaltend einen, mehrere oder alle der folgenden Pipettierparameter: das Gesamtvolumen der zu verteilenden Probe, die Teilvolumina der zu verteilenden Probe und/oder die Anzahl von Abgabeschritten zur Abgabe gleicher Teilvolumina, die Probenaufnahmegeschwindigkeit und/oder eine eventuell davon abweichende Probenabgabegeschwindigkeit, das Volumens eines Umkehrhubs, das Volumen eines Überhubs, ggf. das Volumen und die Wiederholungen von Vorbenetzungsschritten der Pipettierbehälter, insbesondere Pipettenspitzen. Die Dispensierfunktion eignet sich insbesondere zum schnellen Befüllen einer Mikrotiterplatte mit einer Reagenzflüssigkeit und kann z.B. zur Durchführung eines ELISA verwendet werden.

Vorzugsweise betrifft ein Anwendungsszenario das "Automatische Dispensieren" (ADS) einer Probe. Zugeordnete Betriebsparameter sind jeweils vorzugsweise: das Volumen der Einzelprobe, betreffend das Pipettiervolumen während eines von mehreren Abgabeschritten; die Anzahl der Abgabeschritte; die Dauer des Zeitintervalls, gemäß dem die Abgabeschritte automatisch in konstanten Zeitabständen nacheinander durchgeführt werden - das Zeitintervall kann diese Zeitabstände festlegen oder z.B. die Verzögerung zwischen Ende und Beginn aufeinander folgender Abgabeschritte; die Geschwindigkeit bei der Aufnahme der Probe(n); die Geschwindigkeit bei der Abgabe der Probe(n). Diese Dispensierfunktion eignet sich noch komfortabler zum Befüllen einer Mikrotiterplatte, da der Anwender nicht immer wieder einen Abgabeschritt durch Betätigung, z.B. Tastendruck, auslösen muss, sondern die Abgabe nach dem Starten des automatischen Dispensierens zeitgesteuert erfolgt. Die Ausführung des zu einem solchen Pipettierparametersatzes gehörenden Betriebsprogramms kann das automatische Dispensieren unter der Bedingung erfolgen, dass das entsprechende Programm zumindest bei ununterbrochener Betätigung eines Betätigungselementes erfolgt, z.B. bei ununterbrochen gedrückt gehaltener Taste. Dies ist zum Beispiel bei langen Dispensierserien oder Reaktionen, bei denen eine genaue Beachtung eines Zeitfensters erforderlich ist, vorteilhaft. Die automatische Dispensierfunktion eignet sich noch komfortabler zum Befüllen einer Mikrotiterplatte, da der Benutzer einen einzelnen Abgabeschritt hier nicht selbst auslösen muss, sondern dies automatisch erfolgt, was z.B. zur Durchführung eines ELISA verwendet werden kann.

Vorzugsweise betrifft ein Anwendungsszenario das "Pipettieren" (Pip) einer Probe. Zugeordnete Pipettierparameter sind insbesondere: das Volumen der zu pipettierenden Probe; die Geschwindigkeit bei der Aufnahme der Probe; die Geschwindigkeit bei der Abgabe der Probe.

Vorzugsweise betrifft ein Anwendungsszenario das "Pipettieren mit anschließendem Mischen" (P/Mix) einer Probe. Zugeordnete Pipettierparameter sind jeweils vorzugsweise: das Volumen der anzusaugenden und/oder der abzugebenden Probe; das Mischvolumen; die Anzahl der Mischzyklen; die Geschwindigkeit bei der Aufnahme der Probe; die Geschwindigkeit bei der Abgabe der Probe. Die Funktion "Pipettieren mit anschließendem Mischen" empfiehlt sich beispielsweise für das Pipettieren von sehr kleinen Volumina. Wird ein Dosiervolumen < 10 *µ* L gewählt, empfiehlt es sich, dieses in die jeweilige Reaktionsflüssigkeit einzuspülen. Dies ist möglich durch das automatische Starten einer Mischbewegung nach Abgabe der Flüssigkeit. Das Mischvolumen sowie die Mischzyklen werden zuvor definiert. Eine Anwendung für diesen Betriebsmodus ist z.B. die Abgabe einer aufgrund ihrer physikalischen Eigenschaften schwerer als Wasser zu dosierenden Flüssigkeit, deren Reste im Pipettierbehälter, insbesondere der Pipettenspitze, dann mithilfe der bereits vorgelegten Flüssigkeit aus dem Pipettierbehälter, bzw. der Pipettenspitze, gespült wird. Eine weitere Anwendung wäre das sofortige Mischen der abgegebenen Flüssigkeit mit der vorgelegten Flüssigkeit. Vorteilhaft ist dieser Betriebsmodus z.B. beim Zugeben von DNA zu einer PCR-Mischlösung.

Vorzugsweise betrifft ein Anwendungsszenario die "Mehrfachaufnahme" einer Probe, auch bezeichnet als "Umgekehrtes Dispensieren" oder als "ASP" für Aspiration. Zugeordnete Pipettierparameter sind jeweils vorzugsweise: das Volumen der anzusaugenden Probe(n); die Anzahl der Proben; die Geschwindigkeit bei Aufnahme; die Geschwindigkeit bei Abgabe. Die Funktion dient der Mehrfachaufnahme einer Flüssigkeitsmenge und Abgabe der Gesamtmenge. Hierbei ist eine Mehrfachbefüllung des Pipettierbehälters in einem Vorgang nicht vorgesehen. Die Geschwindigkeit ist für alle Proben gleich. In der Ausführung geschieht vorzugsweise folgendes: Ausgehend von der Grundposition nimmt die Pipettiervorrichtung durch Betätigen erster Art der Bedieneinrichtung jeweils ein Teilvolumen auf. Nach Aufnahme des letzten Teilvolumens gibt die Pipettiervorrichtung vorzugsweise eine Warnmeldung aus, die vorzugsweise durch ein Betätigen zweiter Art der Bedieneinrichtung durch den Anwender bestätigt werden muss. Bei der nächsten Betätigung zweiter Art der Bedieneinrichtung wird das Gesamtvolumen wieder abgegeben. Zur Betätigung erster oder zweiter Art weist die Bedieneinrichtung vorzugsweise mindestens zwei Bedienelemente auf, einen zum Eingeben eines Bedienungssignals "erster Art" an die Steuereinrichtung, und einen zum Eingeben eines Bedienungssignals "zweiter Art" an die Steuereinrichtung. Die Bedieneinrichtung kann insbesondere eine Wippe aufweisen, die insbesondere um eine Achse senkrecht zur Längsachse der Pipettiervorrichtung schwenkbar ist, zwischen einer ersten Signal-Auslöse-Position ("Wippe up") zur Betätigung erster Art und einer zweiten Signal-Auslöse-Position ("Wippe down") zur Betätigung zweiter Art.

Vorzugsweise betrifft ein Anwendungsszenario das "Diluting" (Dil) einer Probe, auch bezeichnet als "Verdünnung". Zugeordnete Pipettierparameter sind jeweils vorzugsweise: das Probenvolumen; das Luftblasenvolumen; das Diluentvolumen; die Geschwindigkeit der Aufnahme; die Geschwindigkeit der Abgabe. Das maximale Diluentvolumen = Nennvolumen - (Probe + Luftblase)). Diese Funktion dient der Aufnahme einer Probe und eines Diluents mit Trennung durch eine Luftblase und Abgabe der Gesamtmenge. Die Geschwindigkeit ist für alle Teilvolumina gleich. In der Ausführung geschieht vorzugsweise folgendes: Ausgehend von der Grundposition nimmt die Pipettiervorrichtung zuerst das Diluentvolumen, dann die Luftblase und zum Schluss die Probe auf. Jede Aufnahme wird vorzugsweise separat durch eine Betätigung der Bedieneinrichtung erster Art ausgelöst. Danach wird die Gesamtmenge in Einem abgegeben.

Vorzugsweise betrifft ein Anwendungsszenario das "Sequenzielle Dispensieren" (SeqD) von Proben. Zugeordnete Pipettierparameter sind jeweils vorzugsweise: Anzahl der Proben (vorzugsweise bis zu einer fest vorgegebenen Maximalanzahl Nmax von vorzugsweise 5 <= Nmax <= 16, vorzugsweise Nmax = 10); Einzelvolumen der Einzelproben; Geschwindigkeit der Aufnahme; Geschwindigkeit der Abgabe. Diese Funktion dient dem sequenziellen Dispensieren von Nmax frei wählbaren Volumina, hierbei ist vorzugsweise eine mehrfache Befüllung des Pipettierbehälters nicht vorgesehen. Die Geschwindigkeit ist für alle Proben gleich. Die Anzahl der Proben ist vorzugsweise der führende Parameter für die Eingabe der Einzelvolumen. Die Pipettiervorrichtung muss vorzugsweise bei Eingabe der Volumina immer überprüfen, ob das Maximalvolumen der Pipettiervorrichtungen nicht überschritten wird, ggf. wird eine Warnmeldung ausgegeben. Nach Eingabe aller Parameter nimmt die Pipettiervorrichtung nach Betätigen erster Art der Bedieneinrichtung das Gesamtvolumen auf und nach Betätigen zweiter Art der Bedieneinrichtung jeweils ein Einzelvolumen ab. Alle weiteren Abläufe verhalten sich vorzugsweise wie das normale Dispensieren.

Vorzugsweise betrifft ein Anwendungsszenario das "Sequenzielle Pipettieren" (SeqP) von Proben. Zugeordnete Pipettierparameter sind jeweils vorzugsweise: Anzahl der Proben (vorzugsweise bis zu einer fest vorgegebenen Maximalanzahl Nmax von vorzugsweise 5 <= Nmax <= 15, vorzugsweise Nmax = 10); Einzelvolumen der Einzelproben; Geschwindigkeit der Aufnahme; Geschwindigkeit der Abgabe. Diese Funktion dient dem Pipettieren von maximal Nmax frei wählbaren Volumina, die vor dem Start programmiert werden und in Ihrer Abfolge feststehen. Die Geschwindigkeit ist für alle Proben vorzugsweise gleich. Die Geschwindigkeit kann aber auch unterschiedlich einstellbar sein. Der Ablauf der Funktion entspricht dem Ablauf des Pipettierens. Es werden die vorher eingegebenen Volumina in der programmierten Reihenfolge abgearbeitet. Nach Abgabe wird über die Betätigung eines Bedienelementes, z.B. Tastendruck, entschieden, ob die nächste Probe aufgenommen werden soll oder vor der nächsten erst ein "Blowout", also ein vollständiges, sicheres Ausblasen der im Pipettierbehälter noch enthaltenen Probe mittels Überhub, und/oder ob ein Wechsel des Pipettierbehälters erfolgen soll.

Vorzugsweise betrifft ein Anwendungsszenario das "Reverse Pipettieren" (rPip) von Proben. Zugeordnete Pipettierparameter sind jeweils vorzugsweise: das Volumen der Einzelprobe; die Geschwindigkeit der Aufnahme; die Geschwindigkeit der Abgabe; Aktivierung des Counters. Bei dieser Funktion "rPip" wird mehr als das zu dosierende Volumen aufgenommen. Dies wird erreicht, indem der Kolben vor der Flüssigkeitsaufnahme, nämlich durch Betätigung zweiter Art, d.h., z.B. mittels Tastendruck oder "Wippe nach unten", nach unten gefahren wird, bis in die untere Position eines Blowouts, also eines Überhubs des Kolbens, der über die Stellung des Kolbens bei einem Pipettierhub hinausgeht. Bei Start der Volumenaufnahme nimmt die Pipettiervorrichtung das Volumen des Blowout's und das eingestellte Volumen auf. Um das Spiel im Antrieb in Abgaberichtung heraus zu nehmen, vollzieht die Pipettiervorrichtung einen zusätzlichen Hub, der sofort wieder abgegeben wird. Dies ist dem Dispensieren ähnlich, erfolgt jedoch vorzugsweise unter automatischer Abgabe des Verwerfhubes mit maximaler Geschwindigkeit.

In der Ausführung des Anwendungsszenarios "rPip" geschieht vorzugsweise Folgendes: Erstens der Kolben der Pipettiervorrichtung (oder der Dispenserspitze) fährt automatisch zum Blowout und bleibt in der unteren Position stehen. Zweitens erfolgt eine Betätigung erster Art der Bedieneinrichtung: Kolben fährt um die Blowout-Strecke und um den Hub für das Pipettiervolumen nach oben. Drittens erfolgt eine Betätigung zweiter Art der Bedieneinrichtung: Kolben fährt den Hub für das Pipettiervolumen nach unten und bleibt vor dem Blowout stehen. Viertens erfolgen zwei Betätigungen zweiter Art der Bedieneinrichtung: Kolben führt den Blowout aus und bleibt in der unteren Position stehen. Alternativ zu "viertens" erfolgt eine Betätigung erster Art der Bedieneinrichtung: Kolben fährt den Pipettierhub nach oben. Der Modus "rPip" eignet sich insbesondere zum Pipettieren von Plasma, Seren und anderen Flüssigkeiten mit hohem Proteingehalt. Für wässrige Lösungen eignet sich insbesondere der Modus "Pipettieren". Der Modus "rPip" eignet sich insbesondere für netzmittelhaltige Lösungen, um die Schaumbildung bei der Abgabe in das Zielgefäß zu minimieren. Die Flüssigkeit wird insbesondere mit Überhub (Blowout-Volumen) aufgenommen. Der Überhub gehört hierbei typischerweise nicht zum Abgabevolumen und wird vorzugsweise nicht in das Zielgefäß abgegeben. Insbesondere wenn dieselbe Probe erneut verwendet wird, kann der Überhub in der Spitze verbleiben. Wenn eine andere Flüssigkeit verwendet wird, wird vorzugsweise der Überhub und/oder vorzugsweise der Pipettierbehälter verworfen.

Durch einen Pipettierparametersatz wird vorzugsweise ein Betriebsprogramm, insbesondere ein Steuerprogramm, zur Durchführung des gewünschten Pipettiervorgangs gesteuert. Das Steuerprogramm kann jeweils in Form von elektrischen Schaltkreisen der Steuereinrichtung ausgebildet sein, und/oder durch einen ausführbaren Programmcode ausgebildet sein, der geeignet ist zum Steuern einer Steuereinrichtung, die programmcodesteuerbar ist und vorzugsweise programmierbar ist.

Das System, die Pipettiervorrichtung, ein portables Computergerät oder ein externes Datenverarbeitungsgerät ist vorzugsweise dazu ausgebildet, die vom Benutzer eingegebenen Werte der Pipettierparameter (Pipettierparameterwerte) automatisch zu überprüfen und mit einem erlaubten Bereich des jeweiligen Pipettierparameters zu vergleichen. Liegt der vom Benutzer eingegebene Pipettierparameterwert außerhalb des zulässigen Bereichs, wird die Eingabe entweder nicht akzeptiert, auf dem Bildschirm und/oder akustisch eine Warnung ausgegeben, oder auf eine Default-Wert gesetzt, der z.B. der Minimalwert oder der Maximalwert oder der zuletzt zulässig eingegebene Wert sein kann, oder ein automatisch korrigierter Wert verwendet.

Vorzugsweise weist die Benutzerschnittstelleneinrichtung mindestens einen berührungsempfindlichen oder nicht berührungsempfindlichen Bildschirm auf und/oder vorzugsweise eine Anzahl von zumindest teilweise vordefinierten und in der Pipettiervorrichtung in Form von Anzeigeseitendaten gespeicherten Anzeigeseiten (auch bezeichnet als "Bildschirmseiten"), die in einem, oder verteilt auf mehrere Bildschirme, vorzugsweise bildschirmfüllend angezeigt werden können. Vorzugsweise ist die Bildschirmfläche im Wesentlichen rechteckförmig, möglich auch quadratisch.

Die Datenverarbeitungseinrichtung ist vorzugsweise zur Anzeige einer graphischen Benutzeroberfläche (graphical user interface - kurz *GUI*) in dem Bildschirm (synonym: Display) der Benutzerschnittstelleneinrichtung programmiert. Bei der Beschreibung der GUI wird nachfolgend teilweise die Formulierung "die Datenverarbeitungseinrichtung ist dazu programmiert, dass" weggelassen und nur die GUI und deren Funktionsweise beschrieben, wobei immer gemeint ist, dass die Datenverarbeitungseinrichtung dazu programmiert ist, die entsprechende GUI und deren Funktionsweise zu implementieren. Diese Implementierung ist für den Fachmann eine Routinetätigkeit.

Die GUI beinhaltet zumindest vorzugsweise die Anzeige einer Bildschirmseite (insbesondere: "Homescreen") in dem Bildschirm, in der Pipettierparameter eines Parametersatzes von Pipettierparametern angezeigt werden. Die Datenverarbeitungseinrichtung ist demnach dazu programmiert, dass in dem Bildschirm eine Bildschirmseite angezeigt wird, in der Pipettierparameter eines Parametersatzes von Pipettierparametern angezeigt werden. Der Ausdruck "es wird im Bildschirm ein Pipettierparameter angezeigt" bedeutet, dass im Bildschirm der aktuell eingestellte Wert des Pipettierparameters angezeigt wird. Zudem kann eine Beschreibung des Pipettierparameters angezeigt werden, insbesondere eine Textbeschreibung, ein charakteristisches Piktogramm, oder eine physikalische Einheit der durch den Pipettierparameter beschriebenen Größe, falls der Pipettierparameter eine solche betrifft, z.B. "µL" für ein Volumen. Der Fachmann kann aber auch aus dem Kontext wissen oder lernen, welchem Pipettierparameter ein angezeigter Wert zugeordnet ist, auch wenn eine Beschreibung im Bildschirm fehlt.

Die Datenverarbeitungseinrichtung ist insbesondere dazu programmiert, dass die Bildschirmseite einen Startbildschirm, synonym auch als Homescreen (analog: Homepage bei Internetbrowsern) bezeichnet, anzeigt. Es ist bevorzugt, dass eine Mehrzahl oder Vielzahl von Nutzereingaben an der GUI zu diesem Homescreen zurückführen. Dieser Homescreen kann vorteilhaft dazu benutzt werden, den gesamten Parametersatz eines benutzerdefinierten -oder aus einem vorgespeicherten Anwendungsszenario übernommenen- Pipettiervorgangs anzuzeigen und Einstellungen der zugehörigen Pipettierparameter zu ermöglichen. Der Homescreen wird insbesondere nach dem Einschalten der Benutzerschnittstelleneinrichtung, insbesondere des portablen Computergeräts, angezeigt, insbesondere unmittelbar nach dem Einschalten, wobei möglich ist, dass zeitlich vor dem Homescreen noch rein informative Seiten eingeblendet werden, z.B. ein Firmenlogo, Geräteinformationen etc.. Das Einschalten kann über eine Benutzerbedienung des Displays oder eines Betätigungselementes des portablen Computergeräts bewirkbar sein, oder kann durch die Messung eines Sensors bewirkt sein, z.B. eines Beschleunigungssensors, Bewegungssensors, Annäherungssensors etc. Ein solcher Homescreen hat sich als für die Anwender intuitiv erfassbares Zentrum des Bedienkonzepts der Pipettiervorrichtung erwiesen.

Die Pipettiervorrichtung oder ein dazu externes Datenverarbeitungsgerät, insbesondere das Computergerät, weisen vorzugsweise eine Datenspeichereinrichtung auf. Diese weist vorzugsweise mindestens einen Datenspeicher auf, insbesondere einen Hardware-Datenspeicher, insbesondere nicht-flüchtigen Datenspeicher, insbesondere einen EPROM oder FLASH Speicher. Sie kann auch einen flüchtigen Datenspeicher aufweisen. Die genannte Datenspeichereinrichtung kann aber auch in einem anderen externen Datenverarbeitungsgerät enthalten sein, z.B. einem Server, der Bestandteil des Systems sein kann. Dieser Server implementiert dann insbesondere ein Filehosting, also einen Cloud-Service.

Die Datenverarbeitungseinrichtung kann Bestandteil einer elektrischen Steuereinrichtung des Systems sein, insbesondere der Pipettiervorrichtung, des portablen Computergeräts oder eines externen Datenverarbeitungsgeräts. Das portable Computergerät, insbesondere die elektrische Steuereinrichtung, können jeweils bevorzugt aufweisen: einen Mikrokontroller, eine CPU, Datenspeicher zum Speichern einer Steuersoftware: Die elektrische Steuereinrichtung, auch abgekürzt bezeichnet als Steuerungseinrichtung oder Steuereinrichtung, weist vorzugsweise eine Datenverarbeitungseinrichtung auf, die insbesondere mindestens einen Zentralprozessor (CPU) aufweist. Die Datenverarbeitungseinrichtung kann einen Microcontroller beinhalten. Die Steuereinrichtung weist vorzugsweise einen Microcontroller auf. Die Steuereinrichtung weist vorzugsweise mindestens eine Speichereinrichtung bzw. einen Datenspeicher zur Speicherung von Daten, insbesondere von Pipettierparametern und/oder eines oder mehrerer Computerprogramme bzw. Computerprogrammcodes auf.

Die Steuereinrichtung beinhaltet vorzugsweise mindestens eine Steuerungssoftware bzw. ein Steuerungsprogramm, welches diesen mindestens einen Pipettierparameter verwendet, um mindestens eine Funktion des Pipettiervorgangs oder einen Teil des Pipettiervorgangs oder den Pipettiervorgang automatisch auszuführen. Die Steuerungssoftware bzw. das Steuerungsprogramm wird insbesondere von der Datenverarbeitungseinrichtung der Steuereinrichtung ausgeführt, insbesondere von einer CPU der Datenverarbeitungseinrichtung. Die Steuerungssoftware bzw. das Steuerungsprogramm ist insbesondere in einer Datenspeichereinrichtung des Geräts gespeichert. Diese Datenspeichereinrichtung ist vorzugsweise ein nicht-flüchtiger Speicher.

Das System, insbesondere die Pipettiervorrichtung, ein portables Computergerät oder ein externes Datenverarbeitungsgerät kann eine Sensoreinrichtung aufweisen, z.B. einen Sensor zur Erfassung eines Umgebungsparameters, insbesondere der Temperatur, der Luftfeuchtigkeit oder des Drucks, der für den Kolbenantrieb der Pipettiervorrichtung verwendete Motorstrom. Der Motorstrom kann insbesondere zur Bestimmung der Viskosität der pipettierten Flüssigkeit verwendet werden, und somit zur Identifizierung der Flüssigkeit. Die Sensoreinrichtung kann auch zur Durchführung einer Messung ausgebildet sein, mit dem ein Parameter, insbesondere Pipettierparameter, insbesondere der Typ eines mit der Pipettiervorrichtung verbundenen Pipettierbehälters, insbesondere das maximale Füllvolumen des Pipettierbehälters, insbesondere einer Pipettenspitze, ermittelbar ist. Die Pipettiervorrichtung oder ein externes Datenverarbeitungsgerät kann dazu ausgebildet sein, mindestens einen Pipettierparameter automatisch in Abhängigkeit vom Messwert der Sensoreinrichtung zu bestimmen. Dadurch lässt sich die Optimierung der für ein präzises Pipettieren erforderlichen Pipettierparameter verbessern.

Die Pipettiervorrichtung und/oder ein portables Computergerät und/oder ein externes Datenverarbeitungsgerät wird vorzugsweise netzunabhängig betrieben. Insbesondere kann das jeweilige Gerät mit einer aufladbaren Spannungsquelle versehen sein, beispielsweise einem oder mehreren Akkus. Für diesen Fall kann das Gerät eine mit der aufladbaren Spannungsquelle verbundene Ladeschnittstelle aufweisen.

Eine Kommunikationseinrichtung, insbesondere die erste oder zweite Kommunikationseinrichtung, ist vorzugsweise für einen drahtlosen Datenaustausch über ein Funknetzwerk eingerichtet. Eine Kommunikationseinrichtung, insbesondere die erste oder zweite Kommunikationseinrichtung, ist vorzugsweise für einen Datenaustausch über WLAN, Bluetooth und/oder LoRa-WAN ausgebildet. Die Systemkomponenten tauschen insbesondere mittels derartiger Funktechnologien Daten aus. Die Systemkomponenten -ohne die mindestens eine Pipettiervorrichtung- können in unterschiedlichen Geräten, also insbesondere Gehäusen, angeordnet sein oder können in demselben Gehäuse angeordnet sein, insbesondere dem Gehäuse eines Computergeräts. Die Datenspeichereinrichtung kann insbesondere als Bestandteil eines Servers zur Implementierung eines Filehosting-Service in das System eingebunden sein.

Pipettierbehälter sind Behälter, die geeignet sind, mit einem Verbindungsabschnitt einer handgehaltenen Pipettiervorrichtung verbunden zu werden, um so das Ansaugen von Flüssigkeit in den Pipettierbehälter, das Halten der Flüssigkeit im Pipettierbehälter und die Abgabe daraus zu ermöglichen. Beispiele für Pipettierbehälter sind Pipettenspitzen und Dispenserspitzen.

Pipettenspitzen sind insbesondere Einwegprodukte und bestehen vorzugsweise aus Kunststoff. Je nach benötigtem maximalem Flüssigkeitsvolumen werden unterschiedliche Pipettenspitzen mit der Kolbenhubpipette verwendet. Typische Nennvolumina von handelsüblichen Pipettenspitzen sind z.B. 10 µL, 20 µL, 100 µL, 200 µL, 300 µL, 1000 µL, 1250 µL, 2500 µL, 5 mL, 10 mL (µL: Mikroliter; mL: Milliliter). Eine Pipettenspitze weist in der Regel einen entlang einer Längsachse langgestreckten konusförmigen Behälter auf, der am unteren Ende eine Flüssigkeitsaustauschöffnung aufweist, und der am oberen Ende einen konus- und rohrförmigen, nach oben geöffneten Endabschnitt aufweist, der auf einen als Arbeitskonus ausgebildeten Verbindungsabschnitt der als Luftpolsterpipette ausgebildeten Pipettiervorrichtung insbesondere aufsteckbar ist. Das Einsaugen der Flüssigkeit in die Pipettenspitze erfolgt über einen Unterdruck im Innenraum der Pipettenspitze, der im Fall der als Luftpolsterpipette ausgebildeten Pipettiervorrichtung durch eine Bewegung des als Kolben ausgebildeten Bewegungselements der Pipettiervorrichtung erzeugt wird, indem im Luftraum oberhalb der Probe in der Pipettenspitze ein Unterdruck erzeugt wird. Der Innenraum der Pipettenspitze ist in einer Pipettierposition, auch als Aufsteckposition bezeichnet, in der die Pipettenspitze mit dem Verbindungsabschnitt der Kolbenhubpipette verbunden ist, fluidisch mit dem Pipettierkanal der Kolbenhubpipette verbunden, der über einen in einer hohlzylinderförmigen Kolbenkammer elektrisch bewegbaren Zylinderkolben der Kolbenhubpipette mit dem Unterdruck/Überdruck beaufschlagt wird.

Eine Dispenserspitze wird bei Direktverdrängern eingesetzt, bei denen beim Pipettieren zwischen Kolben und flüssiger Probe im Wesentlichen kein Luftpolster vorliegt. Der Kolben ist hier Bestandteil der Dispenserspitze. Diese weist einen als Kolbenzylinder fungierenden Behälterteil mit einer Behältermündung und eine Behälteröffnung auf, in die der Kolben eingreift, um im Kolbenzylinder verschiebbar angeordnet zu sein und in einer Endposition den gesamten aspirierten Flüssigkeitsinhalt verdrängen zu können. Beim Verbinden der Dispenserspitze mit der als Direktverdränger ausgebildeten Pipettiervorrichtung wird der Behälterteil mit dem Verbindungsabschnitt der Pipettiervorrichtung verbunden, insbesondere durch Aufstecken oder Aufschrauben, und der Kolben koppelt durch eine Kopplungseinrichtung an das Bewegungselement. Dispenserspitzen sind insbesondere Einwegprodukte und bestehen vorzugsweise aus Kunststoff. Je nach benötigtem maximalem Flüssigkeitsvolumen werden unterschiedliche Dispenserspitzen mit dem Direktverdränger verwendet. Typische Nennvolumina von handelsüblichen Dispenserspitzen sind z.B. 100 µL, 200 µL, 500 µL, 1 mL, 2,5 mL, 5 mL, 10 mL, 25 mL, 50 mL.

Die im Rahmen der vorliegenden Erfindung beschriebenen handgehaltenen Pipettiervorrichtungen sind vorzugsweise, aber nicht ausschließlich, handgehaltene computergesteuerte Kolbenhubpipetten mit elektrischem Kolbenantrieb, auch bezeichnet als handgehaltene, elektrische Pipettiervorrichtungen oder handgehaltene, elektrische Kolbenhubpipetten. Die mit dem erfindungsgemäßen System verwendbaren Pipettiervorrichtungen können aber auch mechanische, insbesondere manuell angetriebene, Pipettiervorrichtungen sein. Eine Möglichkeit, dies zu realisieren, wäre die Durchführung eines automatischen Identifikationsverfahrens zur Identifizierung einer Pipettiervorrichtung, insbesondere durch ein Scannen des Barcode oder QR-Codes, der am Gehäuse einer mechanischen Pipettiervorrichtung angebracht sein kann und der als Information Identifikationsdaten (eine ID, z.B. Seriennummer). Diese Informationen lassen sich dann im entsprechenden Methodendatensatz hinterlegen, um zu dokumentieren, welche Pipettiervorrichtung(en) benutzt wurde(n). Die Aufzeichnung der Pipettiervorgänge einer mechanischen Pipettiervorrichtung kann über ein Erfassungssystem, z.B. einen Sensor der Pipettiervorrichtung oder ein Bilderfassungs- und verarbeitungssystem des Systems, ermöglicht werden.

Die Erfindung betrifft auch ein Verfahren zur Anwendung des erfindungsgemäßen Systems, aufweisend die Schritte:
a) in Abhängigkeit von einer Benutzereingabe die Aufzeichnung eines ersten Aufzeichnungsabschnitts der ersten Methodenaufzeichnung zu starten, bei dem eine erste Untermenge der ersten Abfolge von Parametersätzen des ersten Experimentabschnitts aufgezeichnet und als erster Methodendatensatz in der Datenspeichereinrichtung gespeichert wird,
b) in Abhängigkeit von einer Benutzereingabe die erste Methodenaufzeichnung zu pausieren; und
   vorzugsweise: c) in Abhängigkeit von einer Benutzereingabe und den ersten Methoden-Identifikationsdaten den gespeicherten, ersten Methodendatensatz auszuwählen und die das erste Experiment aufzeichnende erste Methodenaufzeichnung zu beenden oder fortzusetzen, wobei im Falle des Fortsetzens die Aufzeichnung eines zweiten Aufzeichnungsabschnitts der ersten Methodenaufzeichnung gestartet wird, bei dem die zweite Untermenge der ersten Abfolge von Parametersätzen des zweiten Experimentabschnitts aufgezeichnet wird.

Weitere bevorzugte Ausgestaltungen des erfindungsgemäßen Verfahrens und des Systems ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1a zeigt in einer schematischen Seitenansicht ein Ausführungsbeispiel einer Pipettiervorrichtung, die mit einem erfindungsgemäßen System der Fig. 1b verwendbar ist.
Fig. 1b zeigt ein erfindungsgemäßes System, mit dem die Pipettiervorrichtung der Fig. 1a verwendbar ist und auf dessen Benutzerschnittstellendisplay die in den Figuren 2a bis 2p und 3a bis 3j gezeigten Bildschirmseiten als Bestandteile einer GUI ausgegeben werden.
Fig. 2a bis 2p zeigen jeweils eine Bildschirmseite, die auf dem portablen Computergerät ausgegeben werden können, das einen Bestandteil des erfindungsgemäßen Systems in Fig. 2 bildet.
Fig. 3a bis 3j zeigen jeweils eine Bildschirmseite, die auf dem portablen Computergerät ausgegeben werden können, das einen Bestandteil des erfindungsgemäßen Systems in Fig. 2 bildet.
Fig. 4 zeigt die Sequenz von Benutzerinteraktionen, die in einer Anwendung des erfindungsgemäßen Systems in dessen beispiehafter Ausgestaltung während eines bzw. mehrerer Experimente mit verschachtelten Aufzeichnungen durchgeführt werden können, bzw. die in einem beispielhaften erfindungsgemäßen Verfahren ausgeführt werden.

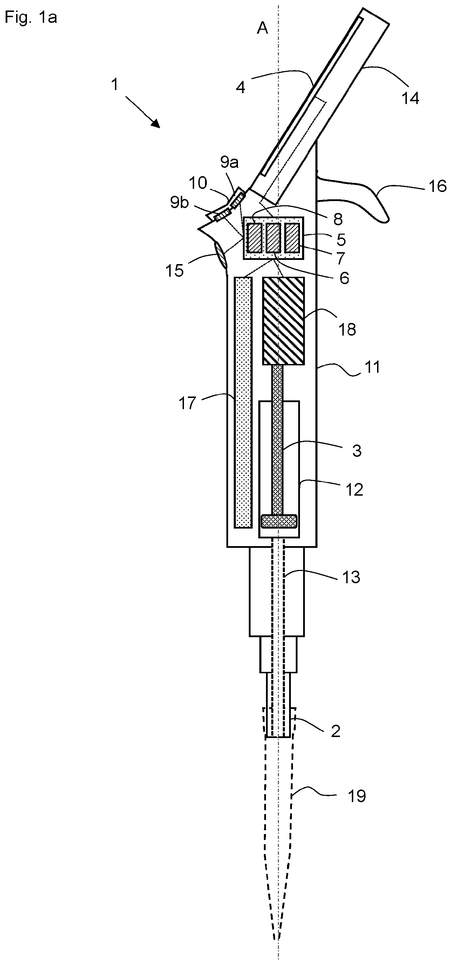

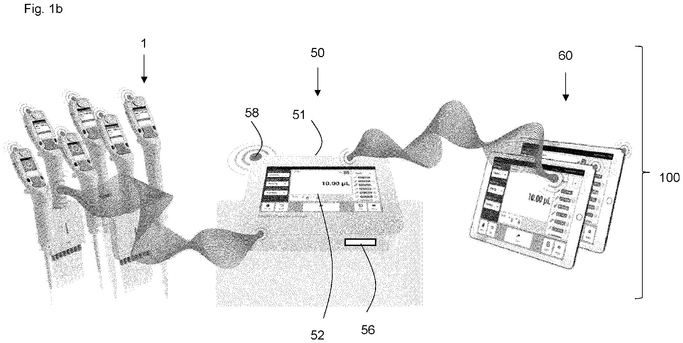

Fig. 1a zeigt eine handgehaltene Pipettiervorrichtung 1 zum Pipettieren mindestens einer flüssigen Probe, hier eine Luftpolsterpipette 1, die, neben optional weiteren ähnlichen Pipettiervorrichtungen, im erfindungsgemäßen System 100 der Figur 1b einsetzbar ist. Der Pipettiervorrichtung 1 ist eine Seriennummer zugeordnet, die der eindeutigen Identifizierung im System dient. Diese Identifikationsdaten sind im Datenspeicher der Pipette gespeichert und vom System lesbar. Diese Pipettiervorrichtung 1 weist auf: einen Verbindungsabschnitt 2 (Arbeitskonus) zum Verbinden mindestens eines Pipettierbehälters 19 aus Fig. 1a, hier einer Pipettenspitze, ein von der Steuereinrichtung 5 elektrisch gesteuertes Bewegungselement, hier der Kolben 3, zum Ansaugen der mindestens einen Probe in den mindestens einen Pipettierbehälter 19, Halten der Probe in dem mindestens einen Pipettierbehälter und Probenabgabe aus dem mindestens einen Pipettierbehälter bei der Durchführung eines Pipettiervorgangs,
einen berührungsempfindlichen Bildschirm 4 auf der Frontfläche 14 A des Kopfabschnitts 14 der Pipettiervorrichtung, zur Eingabe der Parameterwerte von benutzerdefinierbaren Pipettierparametern, wobei ein Parametersatz von Pipettierparametern den Pipettiervorgang vollständig definiert,
wobei die elektrische Steuereinrichtung 5 eine Datenverarbeitungseinrichtung 6 aufweist, die zur Steuerung des Bewegungselements 3 in Abhängigkeit von dem mindestens einen Pipettierparameter programmiert ist, sowie einen Datenspeicher 7 und eine Kommunikationseinheit 8 zur drahtlosen Kommunikation mit einem externen datenverarbeitenden Gerät -insbesondere dem portablen Computergerät 50 aufweist,
mit zwei Bedienelementen (synonym: Betätigungselementen) 9a und 9b, die mittels einer Bedienwippe 10 vom Benutzer betätigbar sind. Mittels eines Betätigungselements kann insbesondere ein Pipettiervorgang vom Benutzer gestartet werden.

Die Kommunikationseinheit 8 ist als "zweite Kommunikationseinrichtung" im Sinne des Anspruchs zu verstehen und insbesondere dazu eingerichtet, mit der entsprechenden (ersten) Kommunikationseinrichtung 58 des portablen Computergeräts 50 Daten auszutauschen. Insbesondere werden von der Pipette 1 von dem portablen Computergerät Konfigurationsdaten empfangen, um die Pipette 1 in einem am Computergerät 50 ausgewählten Betriebsmodus zu betreiben, insbesondere mit den Pipettierparametern, welche die Pipette ebenfalls vom Computergerät 50 erhält, wo die Pipettierparameter zuvor vom Benutzer festgelegt wurden. Insbesondere können die ausgetauschten Daten Informationen darüber beinhalten: einen an der Pipette 1 eingestellten Betriebsmodus, die beim Pipettiervorgang angewandten Pipettierparameter, Anfangs- und/oder Endzeit der Pipettiervorgänge oder deren Teilschritte, Identifikationsdaten -z.B. Seriennummern- der eingesetzten individuellen Pipettiervorrichtungen, Identifikationsdaten des -gegebenenfalls zuvor authentifizierten oder ausgewählten- Benutzers, der in der Regel der ausführende Verantwortliche eines Experiments sein wird, sowie andere Daten, die von anderen am Experiment beteiligten Geräten und/oder Sensoren eines Labors bereitgestellt sein können. Diese Daten können dann als Daten des Methodendatensatzes gespeichert werden.

Die Pipettiervorrichtung weist einen Elektromotor 18 auf, der von einem Akkumulator 17 gespeist wird und der die Kolbenstange bzw. den Kolben 3 innerhalb des Zylinderkolbens 12 bewegt, so dass im Pipettierkanal 13 ein Ansaugdruck oder ein Abgabedruck die Folge ist. Durch die elektronische Steuerung kann präzise dosiert werden. Es ist ein Abwurfknopf 15 vorgesehen, der den Abwurf der Pipettenspitze 19 vom Verbindungsabschnitt 2 mittels einer Abwurfhülse der Pipettiervorrichtung erlaubt. Die Datenverarbeitungseinrichtung ist insbesondere dazu programmiert, im Falle der Aktuierung des Abwurfknopfs 15 einen laufenden Pipettiervorgang abzubrechen.

Ebenfalls in Fig. 1b zu sehen sind weitere externe Geräte 60, die stellvertretend für Tablet-PCs oder Smartphones stehen, mit denen optional die am Computergerät 50 eingestellten Parameter, insbesondere Pipettierparameter, eingestellt oder ausgelesen werden können.

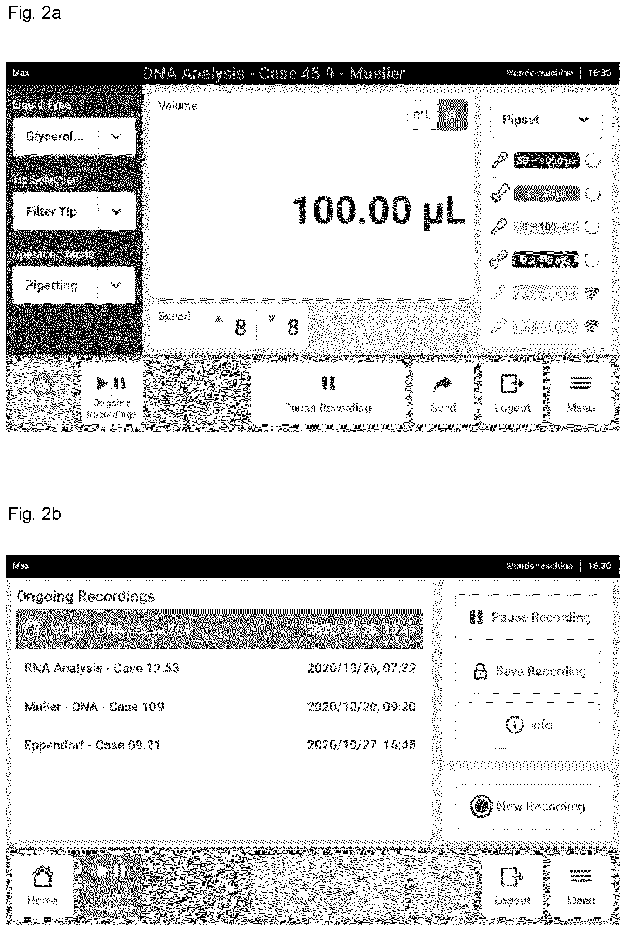

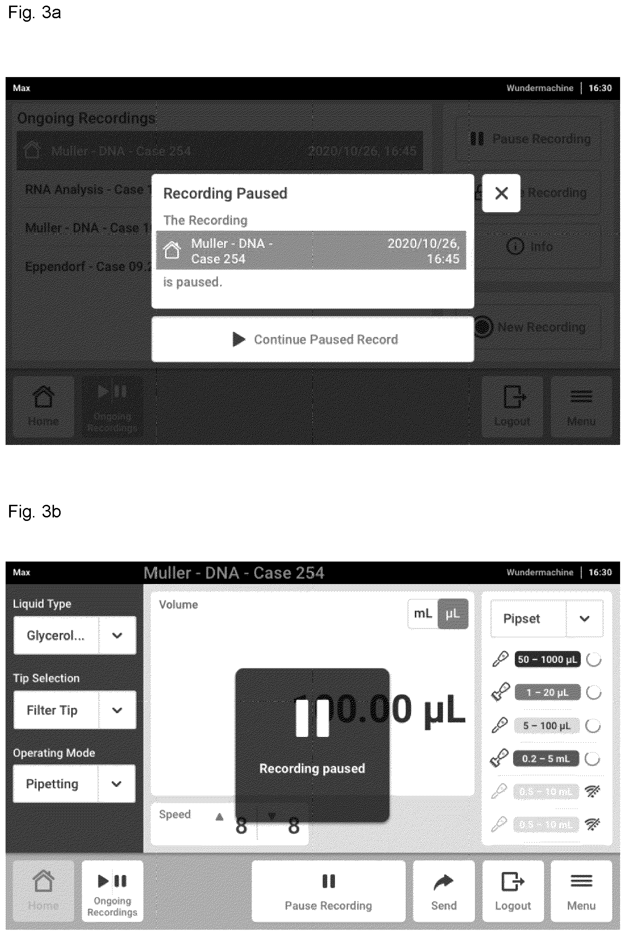

Die Auswahl der Pipettierparameter eines Parametersatzes, der den gewünschten teilautomatisierten Pipettiervorgang definiert, und die Einstellung der Werte dieser Pipettierparameter erfolgt mittels einer GUI am Computergerät 50, wie in Fig. 1b zu sehen. Das Computergerät 50 weist ein Gehäuse 51 auf, in das ein Touchscreen 52 integriert ist. Dieser dient als Benutzerschnittstelleneinrichtung 52 des Systems. Die Datenverarbeitungseinrichtung 56 des Computergeräts 50 ist dazu programmiert, die Methodenaufzeichnung(en) des Systems zu steuern. Die Datenverarbeitungseinrichtung 56 des Computergeräts 50 ist dazu programmiert, im Touchscreen 52 Bildschirmseiten der GUI anzuzeigen, mittels der das System vom Benutzer gesteuert wird. Die entsprechende Steuersoftware ist eine modifizierte Version der Steuersoftware "VisioNize^{®} pipette manager". Solche Bildschirmseiten sind in den Figuren 2a bis 2p und 3a bis 3j dargestellt. Die damit implementierte Funktion der Protokollierung mittels pausierbarer, insbesondere verschachtelbarer, Methodenaufzeichnung wird nachfolgend erläutert.

Innerhalb der Methodenaufzeichnungen wird die Funktion [Process/Run] Records für den VisioNize^{®} pipette manager erweitert. Über einen Bildschirm "Ongoing Recordings" ("Laufende Aufzeichnungen") kann der Benutzer eine Aufzeichnung (auch als Aufnahme bezeichnet) starten, um einen Zeitrahmen zu erstellen, der den Zeitpunkt der Aufzeichnung definiert, sowie von einer speziellen Seite, die über die untere Leiste zugänglichen Seite eine neue Aufzeichnung starten, pausierte Aufzeichnungen bearbeiten oder eine Aufzeichnung speichern.

Die Aufzeichnungen können exportiert werden und ergeben entsprechend den eingestellten Filtern ein PDF.
Eine Methodenaufzeichnung ist (sofern kein Ausnahmefall eintritt) ein Einzelbenutzerszenario.

In einem Homescreen der GUI (gezeigt in Fig. 2a) lassen sich der Betriebsmodus einer verbundenen und einer Methode -identifizierbar über Methodenidentifikationsdaten- zugeordneten Pipette 1 und damit assoziierte Pipttierparameter einstellen, an die Pipette 1 (oder sogar mehrere gleichartige Pipetten 1 oder andere Pipetten) übertragen und diese dadurch konfigurieren.

Die Methodenaufzeichnung ist als Funktion zum Pausieren einer Aufzeichnung auf dem Homescreen verfügbar, vom Bildschirm "Ongoing Recordings" ("laufende Aufzeichnungen"), um eine Aufzeichnung fortzusetzen / zu pausieren / zu speichern / eine neue Aufzeichnung zu starten und aus dem Menü, um die Funktion aufzurufen. Die Bildschirmseite "Menü" zeigt die Schaltfläche "Method Records" ("Methodenaufzeichnungen"). Bei Berührung hat der Benutzer Zugriff auf alle gespeicherten und abgeschlossenen Aufzeichnungen.

Zu Beginn der Laborarbeit kann ein Benutzer beschließen, die nachfolgenden Interaktionen dokumentieren zu wollen. Dies ist vor allem vorteilhaft, um sicherzustellen, dass ein Audit, das möglicherweise zu einem späteren Zeitpunkt stattfindet, erfolgreich bestanden werden kann. Die Funktion der Methodenaufzeichnung unterstützt diese Anforderung. Die Hauptinteraktionen werden vom Startbildschirm aus navigiert.

Auf dem Startbildschirm, der in Fig. 2a gezeigt ist, wird die Information, ob eine Aufzeichnung läuft, in der Statusleiste angezeigt. In der Statusleiste kann einer von drei Stati angezeigt werden:
- Aufzeichnung
- Keine Aufzeichnung
- Pausiert

Die untere Leiste weist eine der Methodenaufzeichnung zugeordnete Schaltfläche auf. Sie zeigt "Ongoing Recordings" mit einem Wiedergabe- /Pausensymbol. Es verhält sich wie das Menü in der unteren Leiste.

Die "Ongoing Recordings" (laufenden Aufnahmen), siehe Bildschirm in Fig. 2b, können über die Schaltfläche "Ongoing Recordings" in der unteren Leiste (sog. "Bottom Bar") aufgerufen werden. Der oberste Listeneintrag ist derjenige, der gerade aufgezeichnet wird. Er ist hervorgehoben und zeigt ein Haus-Symbol - in Anlehnung an "in Gebrauch" auf dem Startbildschirm. Das Datum am Ende des Listeneintrags zeigt das Datum der Erstellung (Jahr/Monat/Datum, Std:Min). Die Listenelemente sind durch eine Trennlinie getrennt.

Die Liste ist dynamisch und verbraucht nur so viel Platz wie benötigt - wenn mehr Aufzeichnungen laufen, als in einen Bildschirm passen, wird eine Bildlaufleiste angezeigt. Die Schaltflächen auf der rechten Seite ermöglichen dem Benutzer, mit den Aufnahmen zu interagieren. Die Schaltflächen sind deaktiviert, solange kein Methodensatz aus der linken Liste ausgewählt ist. "New Recording" ("Neue Aufnahme") ist immer aktiviert und beginnt eine neue Aufzeichnung. Die erste Schaltfläche ist kontextabhängig. Sie kann entweder "Pause Recording" oder "Continue Selected" ("Aufzeichnung pausieren" oder "ausgewählte fortsetzen") bedeuten: Die Schaltfläche "Aufzeichnung pausieren" wird nur angezeigt, wenn die aktive Aufnahme markiert ist. Durch Drücken wird die Aufnahme angehalten (pausiert). Die Schaltfläche "Continue Recording" ("Aufzeichnung fortsetzen") wird nur angezeigt und aktiviert, wenn eine andere Aufnahme als die oberste Aufnahme ausgewählt ist (sie ist deaktiviert, wenn keine Aufnahme ausgewählt ist). "Save Recording"" ("Aufnahme speichern") ermöglicht dem Benutzer das Speichern der Aufnahme. Danach kann sie nicht mehr bearbeitet werden. "Info" zeigt dem Benutzer Informationen über die ausgewählte Aufnahme. Hier kann der Benutzer die Informationen auch bearbeiten (z. B. Titel).

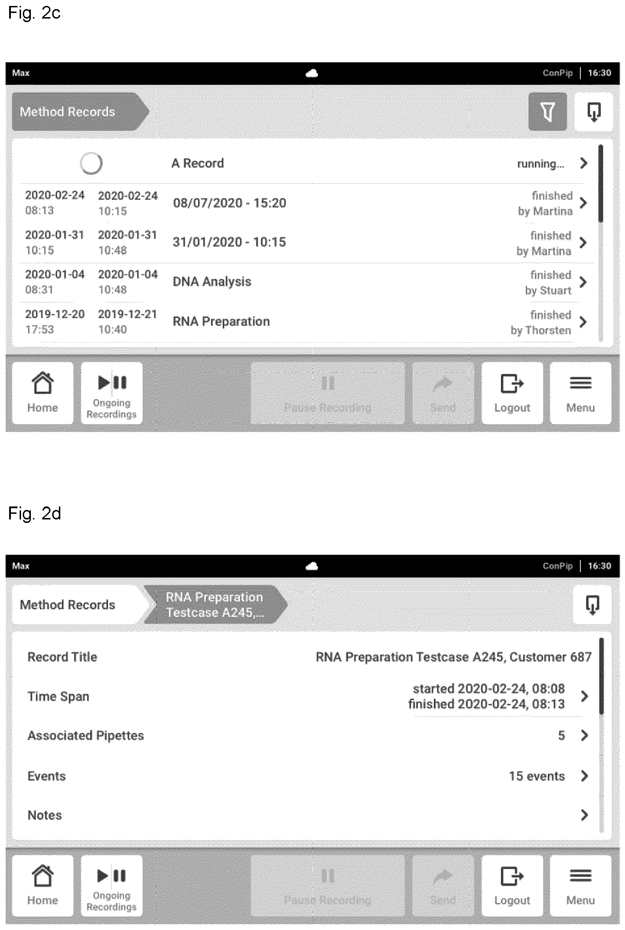

Fig. 2c: Für jede Methodenaufzeichnung (Methodendatensatz) wird ein Start- und Ende-Datum (mit Datum & Uhrzeit) angezeigt. Der Titel der Aufzeichnung wird ebenso angezeigt wie der Status der Aufzeichnung. Der Status einer Aufzeichnung kann einer der folgenden sein:
- "running" ("läuft") ... → zeigt an, dass eine Aufzeichnung gerade durchgeführt wird (mehrere Aufnahmen können als "running" ("laufend") angezeigt werden, z.B. wenn mehrere Benutzer über verschiedene Geräte aufzeichnen - und später können mehrere Aufzeichnungen laufen, wenn ein Benutzer zwischen verschiedenen Aufzeichnungen wechselt, indem er eine Aufzeichnung pausiert und via "continue" eine andere Aufzeichnung fortsetzt oder eine neue Aufzeichnung startet, und während eine Aufnahme läuft, wird die Aktivitätanzeige angezeigt.
- "finished"("abgeschlossen") → eine Aufzeichnung, die beendet und dann gesperrt wurde, so dass sie nicht mehr bearbeitet werden kann.

Unterhalb des Status kann eines der folgenden Informationselemente angezeigt werden:
- "by user" ("von USER") → mit dem Namen des Benutzers oder (wenn deaktivierte Benutzerverwaltung) von "anonymous user"

Es können mehrere Aufzeichnungen in dieser Liste angezeigt werden, wenn die Benutzerverwaltung deaktiviert ist und für den "Unbekannten Benutzer" mehrere Sitzungen aktiv sind, in denen mehrere Aufzeichnungen laufen.
- wenn die Benutzerverwaltung aktiviert ist, wird nur die laufende vom eingeloggten Benutzer auf diesem Gerät angezeigt werden
- wenn die Benutzerverwaltung deaktiviert ist, nachdem Aufzeichnungen bei aktivierter Benutzerverwaltung durchgeführt wurden, wird hier der Name des Benutzers angezeigt werden.

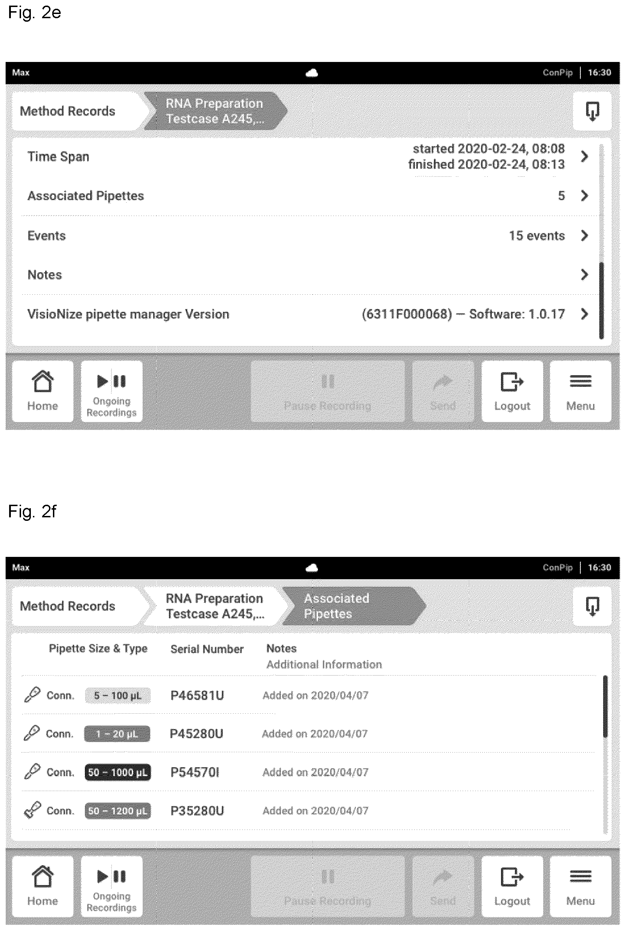

Fig. 2d, 2e: Durch Anklicken des Drill-Down-Indikators neben einer Aufzeichnung gelangt der Benutzer zu einer Übersicht der Aufzeichnung, die folgende Informationen anzeigt:
- Name der Aufzeichnung in der Breadcrumb bar ("aktiver" Teil des Breadcrumbs in blauer Hintergrundfarbe)
- Aufnahmetitel (als Kopfzeile) und der Aufzeichnungs-Titel
- Zeitspanne (als Kopfzeile) mit der Start- & Endzeit der Aufnahme
- Zugehörige Pipetten (Header) mit einer Nummer
   o Alle Pipetten, die für die Aufnahme verwendet wurden (wenn die Pipette aktiv vom Benutzer verwendet wurde) und ein Drill-Down-Indikator
   o nur die Pipetten, die noch Teil der Aufnahme sind, werden für diese Nummer hinzugefügt (Pipetten, die aus der Aufnahme entfernt wurden, sind ausgeschlossen)
   o Entfernte Pipetten werden grau angezeigt am unteren Ende der Liste (mit - falls gegeben- einer Begründung für die Entfernung der Pipette aus der Aufzeichnung)
- Events (Ereignisse)(Kopfzeile) und die Anzahl der Events mit einem Drill-Down-Indikator (die Anzahl erhöht sich, wenn mehr Events auftreten, während eine Aufnahme läuft)
- Notizen (optional → nur, wenn eine Notiz beim Speichern der Aufnahme hinzugefügt wurde)
- VisioNize^{®} pipette manager Version (Kopfzeile) mit der installierten Softwareversion zum Zeitpunkt der Aufnahme

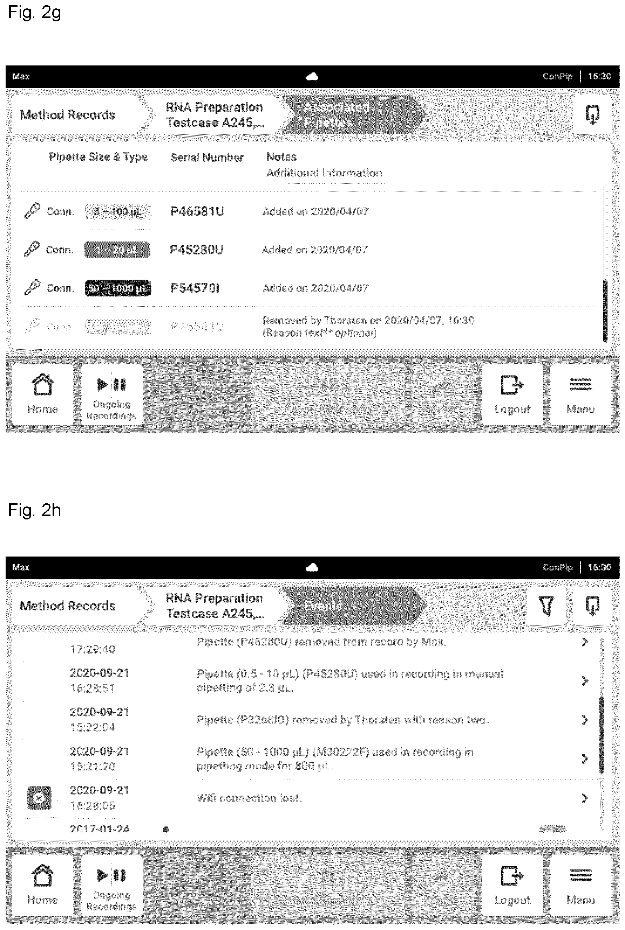

Fig. 2f, 2g: Die Brotkrumenleiste (sog. "Breadcrumb bar") zeigt auch die aktuelle Ebene, die durch die Drilldown-Interaktion auf dem vorherigen Bildschirm erreicht wurde. Durch Klicken auf die Schaltfläche "RNA Preparation Testcase A245" wird die Seite auf der nächsthöheren Hierarchieebene geöffnet. Oben befinden sich auch die Optionen:
- Exportieren

Die zugehörige Seite "Associated Pipettes" zeigt eine Liste mit allen Pipetten, die während der Aufnahme aktiv verwendet wurden. Die Seite ist strukturiert durch eine Kopfzeile mit den folgenden Elementen:
- Pipettengröße und -typ
- Seriennummer
- Notizen (mit "Untertitel"-Informationen")
   ∘ Zusätzliche Informationen

Und eine Liste mit den folgenden Elementen:
- Icon für Pipette (Deutet auf Single- oder Multichannel Pipettentyp hin)
- Volumen-Plakette
- Seriennummer der Pipette
- Zusätzliche Informationen, die bestehen können aus:
   ∘ "Added on" ("Hinzugefügt am") DATUM"
   ∘ "Removed by" ("Entfernt durch") BENUTZER am DATUM + Uhrzeit (opt. Text mit Begründung)" (entfernte Pipetten sind die letzten in der Liste)

Der in diesem Bildschirm ausgelöste Export wird als PDF auf einen USB-Stick exportiert:
- die hier aufgelisteten zugehörigen Pipetten (mit allen angezeigten Informationen) sowie alle gelöschten Pipetten (entsprechend markiert), die Aufzeichnungsnamen, der Benutzer sowie Datum und Uhrzeit der Aufnahme

Die entfernten Pipetten werden in grau dargestellt. Der Volumen-Plakette wird auch in einem deaktivierten Zustand angezeigt.

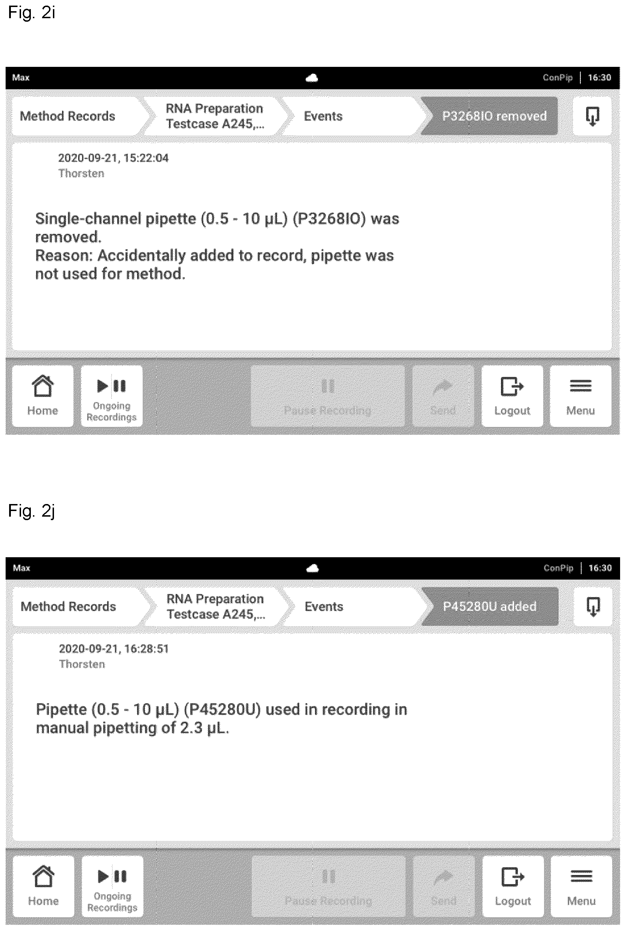

Fig. 2h, 2i, 2j: Die Ereignisse (Events) gruppieren sich um eine Methodenaufzeichnung und sind Bestandteil eines Methodendatensatzes. Das Ereignisdetail sollte zeigen, was "gemacht" wurde, insbesondere ob eine Pipette zur Aufzeichnung hinzugefügt oder entfernt wurde. Es sollte auch der gewählte Grund für das Entfernen der Pipette sowie das Datum und die Uhrzeit angegeben sein, wann dies stattgefunden hat. Auch der Nutzer, der diese Aktion ausgelöst hat, ist hier (gerade beim Entfernen einer Pipette) mit anzugeben.

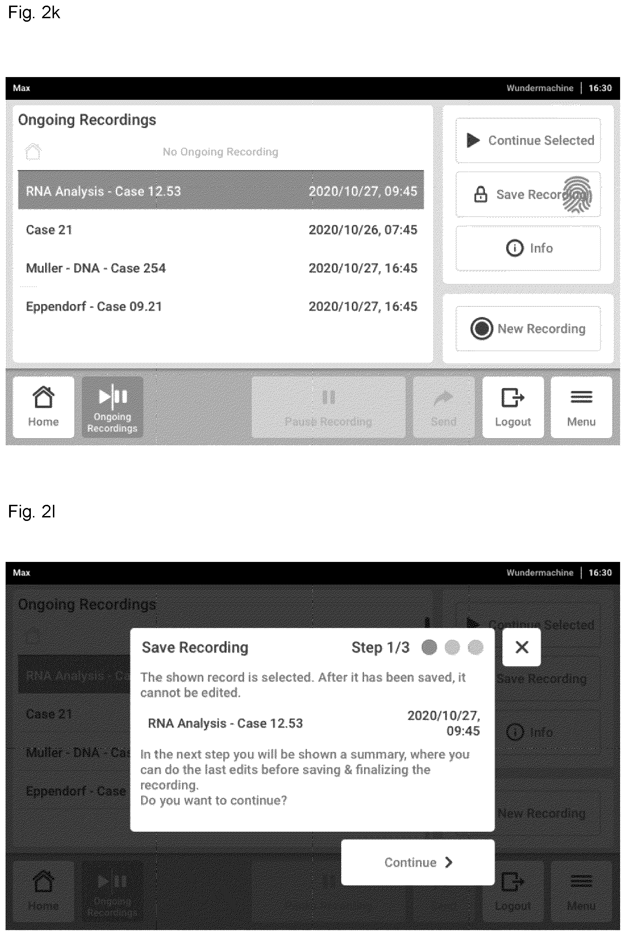

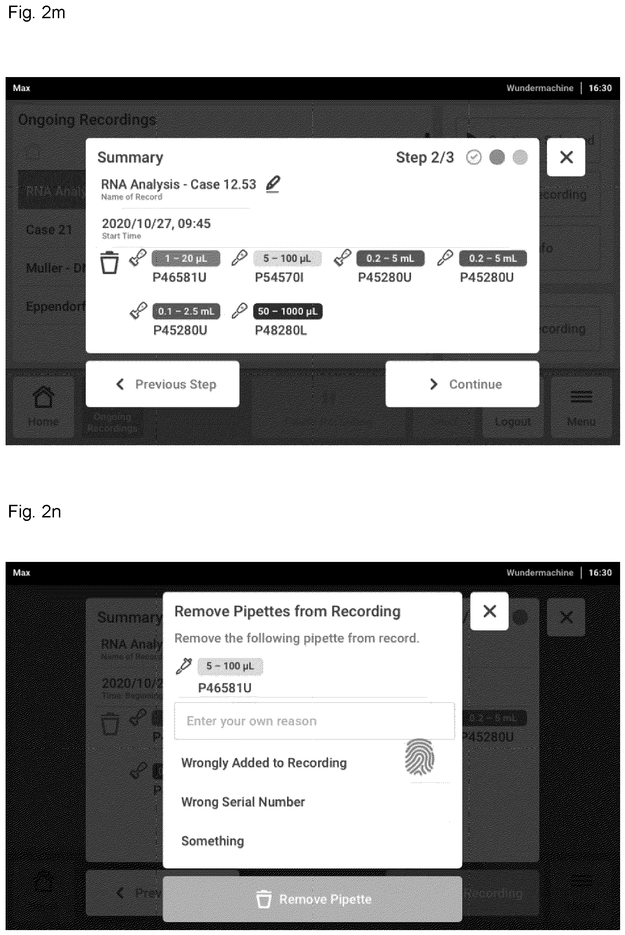

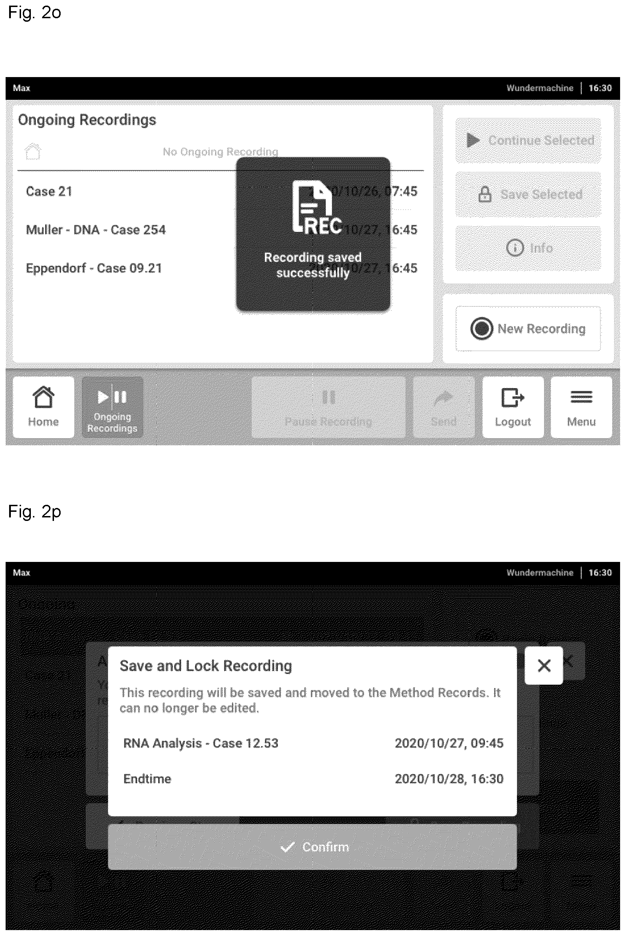

Fig. 2k, 2l, 2m, 2n, 2o, 2p: Der Benutzer kann im Bildschirm mit den laufenden Aufnahmen die Option "Save Recording" ("Aufnahme speichern") wählen. Dies ist immer aktiviert, solange eine Aufnahme aus der Liste ausgewählt ist. Es wird ein Drei-Schritte-Assistent angezeigt. Der Name der Aufzeichnung sowie die Startzeit werden dem Benutzer im Dialog angezeigt. Sobald eine Aufnahme gespeichert ist, kann sie nicht mehr bearbeitet werden - sie wird "gesperrt" ("locked"). Das eingeblendete Overlay umfasst die folgenden Elemente:
- einen Titel,
- einen Schrittzähler für drei Schritte (in Übereinstimmung mit dem Dialog (Assistent))
- einen kurzen Erläuterungstext,
- den Namen der Aufnahme (mit Datum),
- eine "Weiter"-Schaltfläche,
- eine Schaltfläche "Schließen" (X).
(Der gegebenenfalls auf der Bildschirmseite dargestellte Fingerabdruck symbolisiert ein Berühren der entsprechenden Schaltfläche durch den Benutzer).

Der Anwender kann durch Tippen auf die Pipette auswählen, welche Pipetten entfernt werden sollen. Die Pipetten werden als ausgewählt hervorgehoben. Sobald (mindestens eine) Pipette ausgewählt ist, kann der Benutzer auf die Dropdown-Box tippen und ein Overlay wird angezeigt. Die Dropdown-Box wird ansonsten als deaktiviert angezeigt in grau. In Schritt 2/3 wird dem Benutzer eine Zusammenfassung der ausgewählten Aufnahme angezeigt. Die Zusammenfassung zeigt:
- Titel (unter dem die Aufnahme gespeichert ist) mit einer Editiermöglichkeit auf dem Bildschirm
- Zeitpunkt der Aufnahme (Beginn)
- Zugehörige Pipetten; ein Rollbalken wird angezeigt, wenn hier zu viele Pipetten aufgelistet sind; die zugehörigen Pipetten werden als Liste dargestellt, vier Pipetten nebeneinander, und listen das Pipettensymbol, die Plakette, die Seriennummer mit einer Löschoption

Die Informationen der Pipetten sind:
- Icon der Pipette nach Typ (Einkanal, Mehrkanal, mechanisch)
- Volumen-Plakette
- Seriennummer

Der Benutzer kann den Titel ändern (auf dem Bildschirm) oder eine Pipette entfernen. Die Löschtaste ermöglicht Folgendes:
- eine Dropdown-Box wird unterhalb der Pipetten angezeigt
- das Bearbeitungssymbol wird blau
- der Benutzer kann durch Antippen Pipetten auswählen, die aus der Liste entfernt werden sollen

In Schritt 2/3 kann der Benutzer den Titel der Aufnahme bearbeiten (vor dem Speichern) und die zugehörigen Pipetten, die angezeigt werden, löschen. Beim Löschen der Pipetten kann (muss aber nicht) ein Grund ausgewählt werden, warum die Pipette aus der Aufnahme entfernt wurde. Wenn ein Grund angegeben wird, wird der Grund in den Export und Aufnahmedetails angezeigt. Der Benutzer kann auch einen persönlichen Grund angeben. Sobald eine Pipette entfernt wurde, wird sie nicht mehr in der Übersicht in Schritt 2/3 angezeigt. Mehrere Pipetten können auf einmal entfernt werden. Nachdem der Benutzer etwas bearbeitet hat, wird der "Warnung" Pop-up-Dialog dem Benutzer angezeigt, wenn der Assistent zum Schließen ausgewählt wird. Dies dient dazu, um versehentlichen Datenverlust zu vermeiden. Als Schritt 3/3 kann der Benutzer optional eine Notiz zu der Aufnahme eingeben. Bei Auswahl von "Speichern Aufnahme" wird ein Overlay eingeblendet, das weist den Benutzer noch einmal darauf hin, dass die Aufzeichnung gespeichert und anschließend gesperrt wird und nicht bearbeitet werden kann. Wenn dies vom Benutzer akzeptiert wird, wird eine Einblendung im Bildschirm "Laufende Aufzeichnungen" angezeigt, die den Benutzer über das erfolgreiche Speichern der Aufzeichnung informiert. In der Liste, die hier angezeigt wird, fehlt nun die Aufzeichnung, da sie verschoben wurde und nun in den Methodenaufzeichnungen (über das Menü zu erreichen) zu finden ist. Der Grund, Uhrzeit & Datum sowie der Benutzer und welche Pipette (Seriennummer, Typ, Volumen), warum eine Pipette aus der Aufnahme entfernt wurde, wird gespeichert und in jedem Bericht aufgeführt, der aus dieser Aufnahme erstellt wird. Die Informationen werden der Übersicht der "Zugehörigen Pipetten" angezeigt aber die Pipette wird nicht mehr zu den "Zugeordneten Pipetten" gezählt (Numerisch in der Übersichtsliste). Es gibt keine Möglichkeit, eine Pipette dauerhaft aus einer Aufnahme zu löschen - es ist lediglich ein Auslassen aus der Liste, die innerhalb der VisioNize Pipetten-Manager-Software gespeichert ist. Im dritten Schritt ist es möglich, der Aufnahme eine Notiz hinzuzufügen. Das Texteingabefeld vergrößert sich während der Eingabe der Informationen.

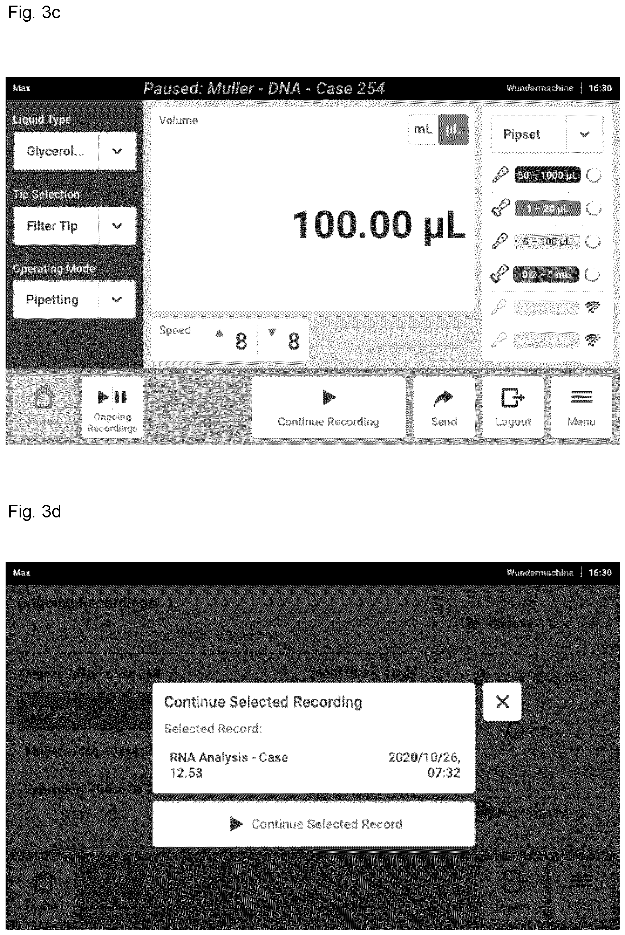

Fig. 3a, 3b, 3c: Der Benutzer kann eine Aufnahme pausieren. Es gibt zwei verschiedene Szenarien und Vorgehensweisen in der GUI. Wenn sich der Benutzer im Bildschirm für laufende Aufnahmen befindet und wählt "Pause Recording" ("Aufnahme anhalten"), wird ein Overlay angezeigt, das dem Benutzer mehrere Optionen bietet. Der Benutzer kann sich für "Continue Paused Record" ("Fortsetzen der pausierten Aufzeichnung") entscheiden, wodurch die Aufnahme fortgesetzt wird; und "unpause" ("Pause aufheben"). Andererseits kann der Benutzer das Overlay mit der "Close"-("Schließen")-Schaltfläche schließen. Durch das Schließen des Overlays wird die Auswahl der Aufzeichnung zurückgenommen und "No ongoing recording" ("Keine laufende Aufzeichnung") wird als oberster Eintrag in dem Bildschirm der laufenden Aufnahmen angezeigt. Die andere Möglichkeit findet sich auf dem Homescreen - hier kann der Benutzer die Aufzeichnung pausieren, indem er auf die Schaltfläche in der unteren Leiste tippt. Wenn diese ausgewählt wird, wird ein Informationspanel eingeblendet, der den Benutzer darüber informiert, dass die Aufzeichnung pausiert ist. In der Statusleiste wird die Aufzeichnung weiterhin angezeigt, jedoch mit dem Zusatz "Paused: Title" ("Pausiert: Titel"). Die Schaltfläche in der unteren Leiste lautet nun "Continue Selected" ("Aufnahme fortsetzen") mit einem Abspielsymbol. Dadurch kann der Benutzer die Aufnahme einfach vom Homescreen aus anhalten und fortsetzen.

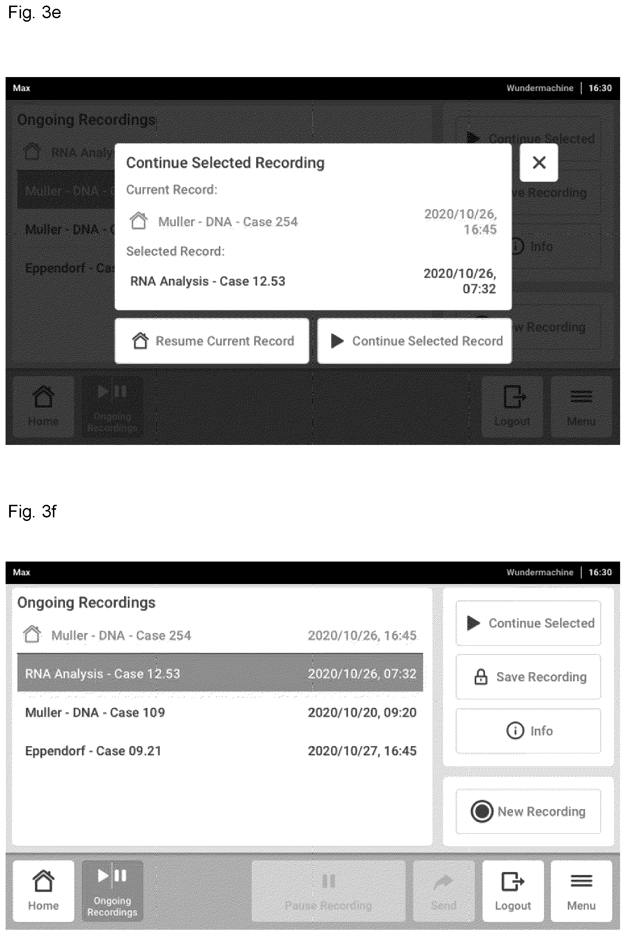

Fig. 3d, 3e, 3f: Wenn eine Aufnahme als Teil der Liste der laufenden Aufzeichnungen angezeigt wird, hat der Benutzer die Möglichkeit, eine Aufzeichnung auszuwählen, um fortzufahren. Wenn eine Aufzeichnung ausgewählt ist, wird ein Overlay angezeigt und je nachdem, ob -oder nicht- sich eine Aufzeichnung gerade im Homescreen befindet, wird das Overlay in einem von zwei Zuständen angezeigt.

Befindet sich keine Aufzeichnung im "Homescreen-Slot", zeigt das Overlay für "Ausgewählte fortsetzen" nur die neu ausgewählte Aufzeichnung und die Schaltflächengruppe im Overlay zeigt nur eine Schaltfläche an. Der Benutzer hat die Möglichkeit, die ausgewählte Aufzeichnung fortzusetzen oder
das Overlay über die Schließen-Schaltfläche zu schließen. Wird das Overlay geschlossen, ist die Aufzeichnung weiterhin ausgewählt aber nicht fortgesetzt. Beim Anklicken von "Continue Selected" ("Ausgewähltes fortsetzen") wird der Benutzer auf den Homescreen gebracht, in dem die fortgesetzte Aufzeichnung läuft, und alle -nun folgenden- Interaktionen sind mit der Aufzeichnung verknüpft.

Befindet sich eine Aufzeichnung im Homescreen-Slot, weist das Overlay unten eine Zwei-Tasten-Gruppe auf und zeigt beide Aufzeichnungen im Overlay an. Die Homescreen Aufzeichnung zeigt das Home-Symbol und wird in blau hervorgehoben - dies dient dazu, die beiden einfach zu unterscheiden und um die gleiche Farbe sowie Icon-Verwendung wiederzuverwenden, wie im Bildschirm für laufende Aufnahmen gezeigt. Wenn der Benutzer die Option "Resume Current Record" ("Aktuelle Aufnahme fortsetzen") auswählt, wird die Aufzeichnung aus dem Homescreen-Slot fortgesetzt. Wenn "Continue Selected Record" ("Ausgewählte Aufnahme fortsetzen") wird die neu ausgewählte Aufnahme fortgesetzt. In beiden Fällen wird der Benutzer auf den Homescreen gebracht, der Name der Aufzeichnung wird in der Statusleiste angezeigt. Beim Schließen des Overlay mit der Schließen-Schaltfläche wird das Overlay geschlossen und die ausgewählte Aufzeichnung ist weiterhin ausgewählt.

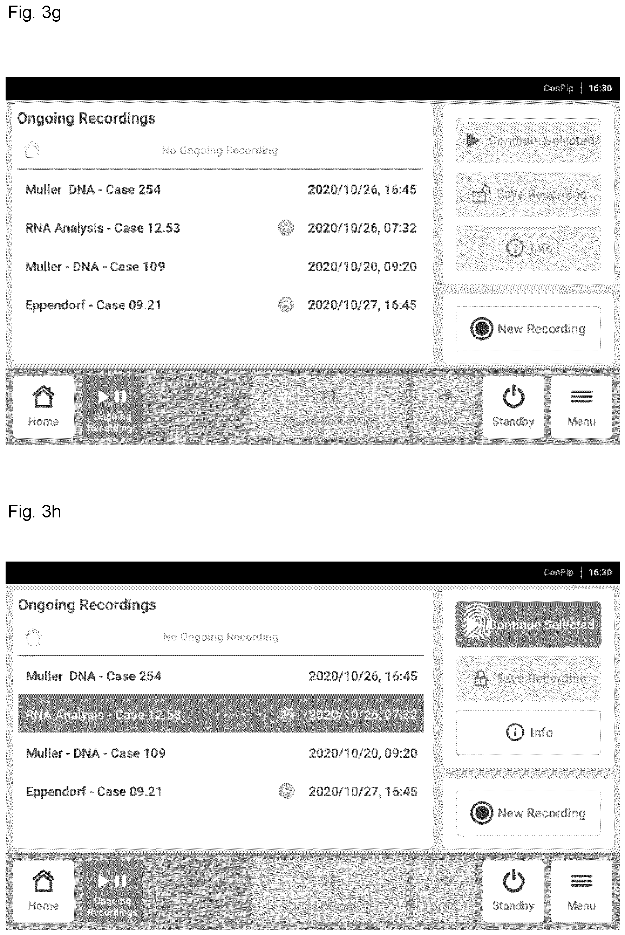

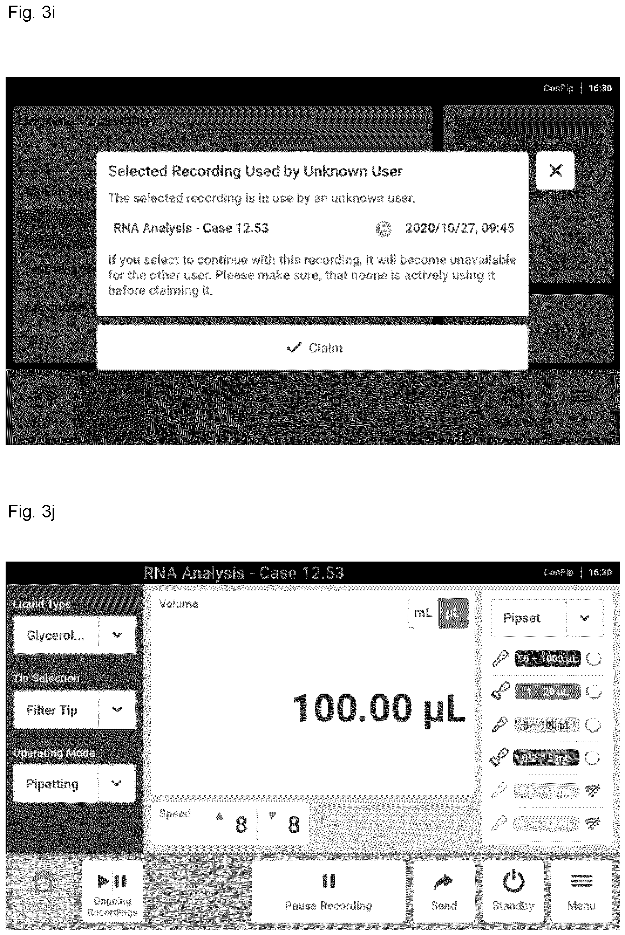

Fig. 3g, 3h, 3i, 3j: Wenn die Benutzerverwaltung deaktiviert ist, werden "sessions" ("Sitzungen") verwendet, um "verschiedene" Benutzersitzungen richtig identifizieren zu können. Dem Benutzer werden alle aktuell laufenden oder pausierten Aufzeichnungen auf der Seite "Laufende Aufzeichnungen" angezeigt. Die Aufzeichnungen, die innerhalb dieser Sitzung auf diesem Gerät aktiv sind, werden ohne einen Hinweis angezeigt, dass ein anderer "Benutzer" mit ihnen verbunden ist. Ein Benutzer kann eine andere Aufzeichnung beanspruchen, um weiter daran arbeiten zu können. Die Seite "Laufende Aufnahmen" und die aufgelisteten Aufzeichnungen zeigen über den Benutzerindikator an, ob eine Aufzeichnung mit einer anderen Sitzung verknüpft ist. Ein Benutzer kann die Aufzeichnung "beanspruchen", indem er sie auswählt (sie wird anschließend hervorgehoben angezeigt) und "Auswahl fortsetzen" wählt. Danach wird ein Popup angezeigt, das den Benutzer darüber informiert, dass diese Aufzeichnung derzeit mit einer anderen Sitzung verbunden ist. Der Name der Aufzeichnung, der Anfangstyp und das Benutzersymbol werden angezeigt. Durch Klicken auf "Beanspruchen" wird die Aufzeichnung zu einer aktiven Aufzeichnung für diese Sitzung, wird in den Homescreen-Slot verschoben und der Benutzer wird zum Homescreen gebracht, auf dem die beanspruchte Aufnahme läuft.

Via einem Sozialprotokoll wird der Benutzer gebeten, zu überprüfen, dass niemand anderes diese Aufzeichnung verwendet, da es keine Möglichkeit gibt, sicherzustellen, dass die Aufnahme tatsächlich frei ist. Ein Datensatz kann nicht zu einem späteren Zeitpunkt zusammengeführt werden, daher ist es wichtig, dass die Benutzer darüber kommunizieren und informiert werden.

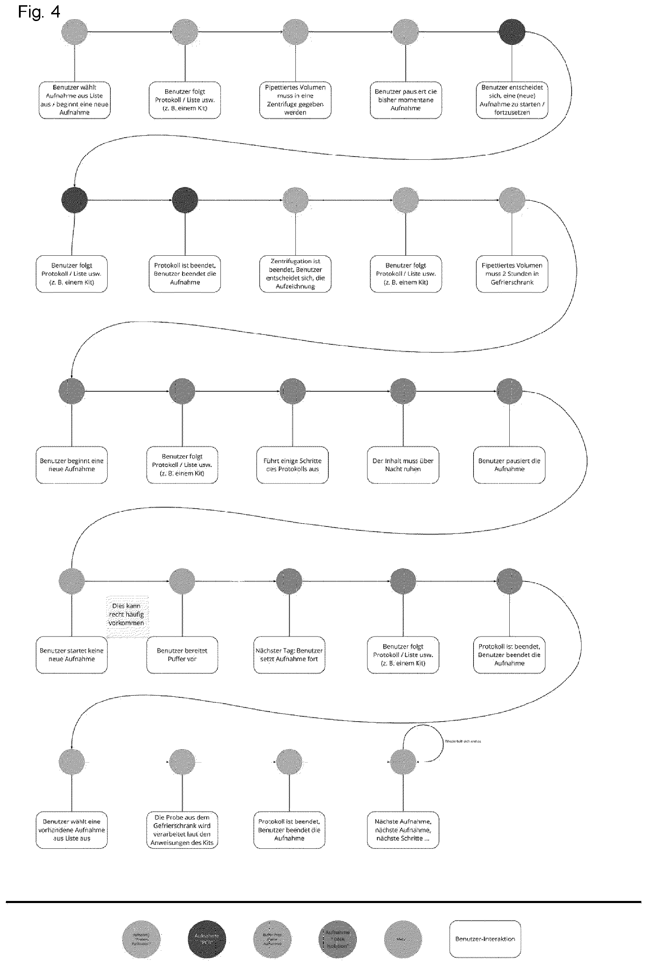

Fig. 4 zeigt die Sequenz von Benutzerinteraktionen, die in einer Anwendung des erfindungsgemäßen Systems in dessen beispiehafter Ausgestaltung während eines bzw. mehrerer Experimente mit verschachtelten Aufzeichnungen durchgeführt werden können, bzw. die in einem beispielhaften erfindungsgemäßen Verfahren ausgeführt werden:
1: Benutzer wählt Aufnahme aus Liste aus / beginnt eine neue Aufnahme
2: Benutzer folgt Protokoll / Liste usw. (z. B. einem Kit)
3: Pipettiertes Volumen muss in eine Zentrifuge gegeben werden
4: Benutzer pausiert die bisher momentane Aufnahme
5: Benutzer entscheidet sich, eine (neue) Aufnahme zu starten / fortzusetzen
6: Benutzer folgt Protokoll / Liste usw. (z. B. einem Kit)
7: Protokoll ist beendet, Benutzer beendet die Aufnahme
8: Zentrifugation ist beendet, Benutzer entscheidet sich, die Aufzeichnung
9: Benutzer folgt Protokoll / Liste usw. (z. B. einem Kit)
10: Pipettiertes Volumen muss 2 Stunden in Gefrierschrank
11: Benutzer beginnt eine neue Aufnahme (bis Schritt 12: Dies kann recht häufig vorkommen)
12: Benutzer bereitet Puffer vor
13: Nächster Tag: Benutzer setzt Aufnahme fort
14: Protokoll / Liste usw. (z. B. einem Kit)
15: Protokoll ist beendet, Benutzer beendet die Aufnahme
16: Benutzer wählt einevorhandene Aufnahme aus Liste aus
17: Die Probe aus dem Gefrierschrank wird verarbeitet laut den Anweisungen des Kits
18: Protokoll ist beendet, Benutzer beendet die Aufnahme
19: Nächste Aufnahme, nächste Aufnahme, nächste Schritte ...
Schleife bei Schritt 19: Wiederholt sich endlos

Farblegende zu Fig. 4:
türkis: Aufnahme (Aufnahme=Aufzeichnung) "Protein Purification"
violett: Aufnahme "PCR"
orange: Buffer Prep (keine Aufnahme)
grün: Aufnahme "DNA Isolation"

## Patentansprüche

1. System zur Aufzeichnung von Pipettiervorgängen in benutzerdefinierten Experimenten, bei denen mindestens eine, handgehalten verwendbare, Pipettiervorrichtung zum Pipettieren mindestens einer Probe verwendet wird, wobei ein Parametersatz von Pipettierparametern einen programmierbaren Pipettiervorgang der Pipettiervorrichtung definiert, und wobei ein Experiment eine Methode beinhaltet, die durch eine Abfolge von Pipettiervorgängen definiert wird,
wobei das System beinhaltet:
• eine Benutzerschnittstelleneinrichtung mit einem Display, insbesondere einem Touchscreen,
• eine Datenverarbeitungseinrichtung,
• eine Datenspeichereinrichtung,
• eine erste Kommunikationseinrichtung für den drahtlosen Datenaustausch zwischen der Datenverarbeitungseinrichtung und einer zweiten Kommunikationseinrichtung mindestens einer Pipettiervorrichtung,
wobei die Datenverarbeitungseinrichtung dazu programmiert ist,
• mittels der ersten Kommunikationseinrichtung nach der Durchführung mindestens eines, einer ersten Methode zugeordneten, Pipettiervorgangs von der mindestens einen zweiten Kommunikationseinrichtung Aufzeichnungsdaten zu empfangen, die die Parameterwerte enthalten, anhand derer der mindestens eine Pipettiervorgang von mindestens einer Pipettiervorrichtung konfiguriert und durchgeführt wurde,
• mittels Speichern der Aufzeichnungsdaten als erstem Methodendatensatz in der Datenspeichereinrichtung eine erste Methodenaufzeichnung durchzuführen, der erste Methoden-Identifikationsdaten zugeordnet sind,
• die Durchführung der ersten Methodenaufzeichnung in Abhängigkeit von einer an der Benutzerschnittstelleneinrichtung erfolgenden Benutzereingabe zu pausieren, und
• die pausierte, erste Methodenaufzeichnung in Abhängigkeit von einer an der Benutzerschnittstelleneinrichtung erfolgenden Benutzereingabe fortzusetzen, oder die pausierte, erste Methodenaufzeichnung in Abhängigkeit von einer an der Benutzerschnittstelleneinrichtung erfolgenden Benutzereingabe zu beenden.

2. System gemäß Anspruch 1, wobei die Datenverarbeitungseinrichtung dazu programmiert ist, die erste Methodenaufzeichnung fortzusetzen, indem
in Abhängigkeit von der Benutzereingabe und den ersten Methoden-Identifikationsdaten der während eines ersten Aufzeichnungsabschnitts der ersten Methodenaufzeichnung gespeicherte, erste Methodendatensatz ausgewählt wird und die Aufzeichnung eines zweiten Aufzeichnungsabschnitts der ersten Methodenaufzeichnung gestartet wird, insbesondere
indem mittels der ersten Kommunikationseinrichtung nach der Durchführung mindestens eines weiteren Pipettiervorgangs von der mindestens einen zweiten Kommunikationseinrichtung weiterer Aufzeichnungsdaten empfangen werden, die die Parameterwerte enthalten, anhand derer der mindestens eine weitere Pipettiervorgang konfiguriert und durchgeführt wurde, und indem mittels Hinzufügen der weiteren Aufzeichnungsdaten zu dem ersten Methodendatensatz und deren Speichern in der Datenspeichereinrichtung die erste Methodenaufzeichnung fortgesetzt wird.

3. System gemäß Anspruch 1 oder 2, das aufweist:
mindestens eine handgehaltene Pipettiervorrichtung zur Durchführung mindestens eines durch die Parameterwerte mindestens eines Parametersatzes von Pipettierparametern definierten Pipettiervorgangs, die eine zweite Kommunikationseinrichtung für den drahtlosen Datenaustausch zwischen der handgehaltenen Pipettiervorrichtung und der Datenverarbeitungseinrichtung aufweist, wobei
die mindestens eine Pipettiervorrichtung insbesondere aufweist:
• einen Verbindungsabschnitt zum Verbinden mindestens eines Pipettierbehälters;
• ein elektrisch gesteuertes Bewegungselement zum Ansaugen mindestens einer Probe in den mindestens einen Pipettierbehälter, Halten der Probe in dem mindestens einen Pipettierbehälter und Probenabgabe aus dem mindestens einen Pipettierbehälter bei der Durchführung eines Pipettiervorgangs; und
• eine elektrische Steuereinrichtung, die zur Steuerung des Bewegungselements in Abhängigkeit von mindestens einem Pipettierparameter eingerichtet ist, und/oder
das System ein, insbesondere portables, Computergerät beinhaltet, das aufweist:
• diese Benutzerschnittstelleneinrichtung,
• diese Datenverarbeitungseinrichtung,
• diese erste Kommunikationseinrichtung, und
• optional: diese Datenspeichereinrichtung.

4. System gemäß einem der vorangehenden Ansprüche, wobei die Datenverarbeitungseinrichtung dazu programmiert ist,
nach dem Beginn und während der Aufzeichnungspause der ersten Methodenaufzeichnung mittels der Benutzerschnittstelleneinrichtung mindestens einen weiteren Parametersatz mindestens eines weiteren Pipettiervorgangs zu erfassen, der keinen Bestandteil der ersten Methode bildet, und wobei die mindestens eine Pipettiervorrichtung, insbesondere deren Steuereinrichtung, dazu eingerichtet ist, während dem Pausieren der ersten Methodenaufzeichnung mindestens einen Pipettiervorgang, insbesondere ein Bewegungselement der Pipettiervorrichtung, in Abhängigkeit von dem mindestens einen weiteren Parametersatz zu steuern.

5. System gemäß einem der vorangehenden Ansprüche und Anspruch 2, wobei die Datenverarbeitungseinrichtung dazu programmiert ist, um, nach dem Pausieren der ersten Methodenaufzeichnung vor dem Start der Aufzeichnung des zweiten Aufzeichnungsabschnitts der ersten Methodenaufzeichnung, eine zweite Methodenaufzeichnung durchzuführen, der zweite, von den ersten Methoden-Identifikationsdaten verschiedene, Methoden-Identifikationsdaten zugeordnet sind,
insbesondere indem die Datenverarbeitungseinrichtung dazu programmiert ist,
• mittels der ersten Kommunikationseinrichtung, nach der Durchführung mindestens eines, einer zweiten Methode zugeordneten, Pipettiervorgangs, von der mindestens einen zweiten Kommunikationseinrichtung Aufzeichnungsdaten zu empfangen, die Parameterwerte enthalten, anhand derer der mindestens eine Pipettiervorgang der zweiten Methode konfiguriert und durchgeführt wurde,
• mittels Speichern dieser Aufzeichnungsdaten als zweitem Methodendatensatz in der Datenspeichereinrichtung die zweite Methodenaufzeichnung durchzuführen,
• die Durchführung der zweiten Methodenaufzeichnung in Abhängigkeit von einer an der Benutzerschnittstelleneinrichtung erfolgenden Benutzereingabe zu pausieren, und
• die pausierte, zweite Methodenaufzeichnung in Abhängigkeit von einer an der Benutzerschnittstelleneinrichtung erfolgenden Benutzereingabe fortzusetzen, oder die pausierte, zweite Methodenaufzeichnung in Abhängigkeit von einer an der Benutzerschnittstelleneinrichtung erfolgenden Benutzereingabe zu beenden.

6. System gemäß Anspruch 5, welches eine bei der ersten Methode zu pipettierende erste Probe beinhaltet und eine bei der zweiten Methode zu pipettierende zweite Probe beinhaltet, die bei der ersten Methode nicht verwendet wird.

7. System gemäß einem der vorangehenden Ansprüche, wobei die Datenverarbeitungseinrichtung dazu programmiert ist, auf dem Display der Benutzerschnittstelleneinrichtung
* eine Bildschirmseite mit einer Schaltfläche anzuzeigen, deren Betätigen bzw. Berühren eine Methodenaufzeichnung startet.

8. System gemäß einem der vorangehenden Ansprüche, wobei die Datenverarbeitungseinrichtung dazu programmiert ist, auf dem Display der Benutzerschnittstelleneinrichtung
* eine Bildschirmseite anzuzeigen, in welcher der Titel mindestens einer laufenden, also gestarteten und nicht beendeten, Methodenaufzeichnung angezeigt wird, insbesondere in welcher die Titel mehrerer laufender Methodenaufzeichnungen aufgelistet sind,
* mindestens ein Informationsfeld anzuzeigen, das den Status der entsprechenden Methodenaufzeichnung anzeigt, der beinhalten kann: Aufzeichnung ist pausiert oder Aufzeichnung läuft oder Aufzeichnung ist beendet.

9. System gemäß einem der vorangehenden Ansprüche, wobei die Datenverarbeitungseinrichtung dazu programmiert ist, auf dem Display der Benutzerschnittstelleneinrichtung
* eine Bildschirmseite anzuzeigen, in welcher der Titel mindestens einer laufenden Methodenaufzeichnung angezeigt wird, insbesondere in welcher die Titel mehrerer laufender Methodenaufzeichnungen aufgelistet sind,
* mindestens eine Schaltfläche anzuzeigen, deren Betätigen bzw. Berühren die mindestens eine, mit der Schaltfläche assoziierte, zuvor pausierte Methodenaufzeichnung auswählt;
* und eine Schaltfläche anzuzeigen, deren Betätigen bzw. Berühren die ausgewählte pausierte Methodenaufzeichnung fortsetzt, insbesondere während mindestens eine andere Methodenaufzeichnung pausiert ist.

10. System gemäß einem der vorangehenden Ansprüche, wobei die Datenverarbeitungseinrichtung dazu programmiert ist, auf dem Display der Benutzerschnittstelleneinrichtung
* eine Bildschirmseite anzuzeigen, in welcher der Titel mindestens einer laufenden Methodenaufzeichnung angezeigt wird, insbesondere in welcher die Titel mehrerer laufender Methodenaufzeichnungen aufgelistet sind,
* mindestens eine Schaltfläche anzuzeigen, deren Betätigen bzw. Berühren die mindestens eine, mit der Schaltfläche assoziierte, zuvor pausierte Methodenaufzeichnung auswählt und die Anzeige von Informationen über diese Methodenaufzeichnung veranlasst, die insbesondere Informationen enthält über, jeweils vorzugsweise: die Ereignisse einer Methode, insbesondere deren zeitliche Sequenz, Zeitdaten der Methode, insbesondere Zeitstempel eines oder mehrerer Ereignisse, insbesondere des Beginns, des Pausierens, und/oder des Endes der Methode, den Benutzer einer Methode, die bei der Methode verwendeten und/oder nicht verwendeten Pipettiervorrichtungen.

11. System gemäß einem der vorangehenden Ansprüche, wobei die Datenverarbeitungseinrichtung dazu programmiert ist, mindestens einen Methodendatensatz mindestens einer beendeten Methodenaufzeichnung als Datei bereitzustellen, die auf ein externes Datenverarbeitungsgerät übertragbar ist, oder deren Inhalt ausgebbar ist durch Drucken, Anzeige auf dem Bildschirm, oder Übertragen zu einem externen Datenverarbeitungsgerät des Systems oder einem Server, der nicht Bestandteil des Systems ist.

12. System gemäß einem der vorangehenden Ansprüche, wobei die Datenverarbeitungseinrichtung dazu programmiert ist,
• mittels von der Benutzerschnittstelleneinrichtung Parameterwerte benutzerdefinierbarer Pipettierparameter mindestens eines Parametersatzes zu erhalten, die den mindestens einen, mittels der mindestens einen Pipettiervorrichtung durchzuführenden, Pipettiervorgang einer Methode, insbesondere ersten, zweiten, und noch weiterer Methoden, definieren,
• mittels der ersten Kommunikationseinrichtung diese Parameterwerte des mindestens einen Parametersatzes an die mindestens eine handgehaltene Pipettiervorrichtung zu übertragen, um deren Konfiguration mittels dieser Parameterwerte zur Durchführung mindestens eines Pipettiervorgangs der Methode zu ermöglichen.

13. System gemäß einem der vorangehenden Ansprüche und Anspruch 3, wobei die mindestens eine Pipettiervorrichtung eine zweite Datenverarbeitungseinrichtung aufweist, die dazu programmiert ist,
• mittels der zweiten Kommunikationseinrichtung die Parameterwerte des mindestens einen Parametersatzes zu erhalten, um die Pipettiervorrichtung mittels dieser Parameterwerte zur Durchführung mindestens eines Pipettiervorgangs der Methode zu konfigurieren,
• den mindestens einen, mittels der Parameterwerte konfigurierten Pipettiervorgang zu starten, insbesondere durch Ermitteln eines durch die Betätigung eines Betätigungselements manuell erzeugten Startsignals, und
• nach Beendigung oder Abbruch des mindestens einen, mittels der Parameterwerte konfigurierten Pipettiervorgang die zur Konfiguration dieses Pipettiervorgangs verwendeten Parameterwerte als Aufzeichnungsdaten an die erste Kommunikationseinrichtung zu übertragen.

14. System gemäß einem der vorangehenden Ansprüche, wobei die Datenverarbeitungseinrichtung dazu programmiert ist, während einer Aufzeichnungspause der ersten Methodenaufzeichnung die von der zweiten Kommunikationseinrichtung an die erste Kommunikationseinrichtung übertragenen Daten, insbesondere Aufzeich-nungsdaten, nicht als Bestandteil der ersten Methodenaufzeichnung im ersten Methodendatensatz zu speichern,
und/oder insbesondere die während der Aufzeichnungspause der ersten Methodenaufzeichnung die von der zweiten Kommunikationseinrichtung an die erste Kommunikationseinrichtung übertragenen Daten, insbesondere Aufzeichnungsdaten, als Bestandteil einer anderen, insbesondere einer zweiten, Methodenaufzeichnung in einem, dieser zugeordneten, weiteren, insbesondere zweiten, Methodendatensatz zu speichern.

15. Verfahren zur Anwendung des Systems gemäß einem der vorangehenden Ansprüche, aufweisend die Schritte:
a) in Abhängigkeit von einer Benutzereingabe die Aufzeichnung eines ersten Aufzeichnungsabschnitts der ersten Methodenaufzeichnung zu starten, bei dem eine erste Untermenge der ersten Abfolge von Parametersätzen des ersten Experimentabschnitts aufgezeichnet und als erster Methodendatensatz in der Datenspeichereinrichtung gespeichert wird,
b) in Abhängigkeit von einer Benutzereingabe die erste Methodenaufzeichnung zu pausieren; und
c) in Abhängigkeit von einer Benutzereingabe und den ersten Methoden-Identifikationsdaten den gespeicherten, ersten Methodendatensatz auszuwählen und die das erste Experiment aufzeichnende, erste Methodenaufzeichnung fortzusetzen, indem die Aufzeichnung eines zweiten Aufzeichnungsabschnitts der ersten Methodenaufzeichnung gestartet wird, bei dem die zweite Untermenge der ersten Abfolge von Parametersätzen des zweiten Experimentabschnitts aufgezeichnet wird.
